(19) Europäisches Patentamt / European Patent Office / Office européen des brevets

(11) EP 2 369 000 A1

(12) **EUROPEAN PATENT APPLICATION**

(43) Date of publication:
**28.09.2011 Bulletin 2011/39**

(51) Int Cl.:
***C12N 15/62*** *(2006.01)* ***C12N 15/79*** *(2006.01)*

(21) Application number: **10382058.5**

(22) Date of filing: **16.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicant: **3T-Science, S.L.**
**41089 Dos Hermanas - Sevilla (ES)**

(72) Inventor: **O'Connor, Kevin James**
**41701 Dos Hermanas - Sevilla (ES)**

(74) Representative: **ABG Patentes, S.L.**
**Avenida de Burgos 16D**
**Edificio Euromor**
**28036 Madrid (ES)**

# (54) Production of peptides and proteins by accumulation in mitochondria

(57) The invention relates to the production of recombinant peptides and proteins in eukaryotic cells and organisms as host systems after transformation of the host with an appropriate vector. A target polypeptide is fused to a synthetic mitochondrial localising sequence and expressed as a fusion product, said fusion product being driven to the mitochondria mediated by the mitochondrial localisation sequence. There the fusion polypeptide is broken to release the product of interest and the mitochondrial localising sequence; the mitochondrial localisation sequence is specifically removed enzymatically by mitochondrial processing enzymes. The target polypeptide is then stored inside the mitochondria and accumulates in the host cell or organism. The isolation and accumulation within the mitochondria allows for protection for the product of interest from the cellular environment, and also for protection of the cell from the product of interest.

EP 2 369 000 A1

## Description

### Field of the Invention

The invention relates to the production of peptides and proteins of interest in a eukaryotic host system by accumulation thereof in a mitochondria based on the use of a mitochondrial targeting sequence to deliver and accumulate said peptide or protein of interest in mitochondria of said host system. The invention also relates to nucleic acid molecules encoding such products and to the use of said nucleic acid molecules in the manufacture of constructs and vectors for transforming eukaryotic host systems.

### Background of the Invention

Efficient expression, accumulation and recovery of recombinant eukaryotic proteins in their native conformations are difficult to achieve. High costs associated with eukaryotic cell-based manufacturing systems are in some cases limiting the advance of biopharmaceutical production. These systems are generally either highly capital-intensive mammalian cell cultures where the proteins are secreted into the media, or require an inefficient, complex and energy dependent refolding from bacterially expressed proteins in the form of insoluble inclusion bodies. According to knowledgeable sources there is still no generic process for producing proteins, with a large body of data gathered for one type of protein i.e. antibodies, all will be different for other proteins [1]. The data for antibody production therefore cannot be transferred to other proteins or cell types other than mammalian cells.

As most data comes from antibody production, it is worth noting that from 1986 to 2004, protein expression has gone up dramatically from 50 mg/L to 4.7 g/L with the main contributor being a massive increase in the number of cells, thanks to media improvement [1]. These improvements have led to a huge increase in the expression (upstream) but not in the purification (downstream). It is in the downstream where energy needs to be focused as it accounts for 75% of manufacturing costs which, in turn, account for 1525% of cost of goods [1]. Significant improvements in the purification phase will have its origins in new and novel technologies and not in the established techniques [2]. Over the past decade, these systems have been used to produce several products and potential therapeutic proteins. Improvements continue to be made, not only in how the proteins are expressed but also in how the end products are obtained. As improvements in expression are realized, cost-saving measures will increasingly focus on downstream processing. Improvements are needed to meet these demands and in downstream processing the main parameters are capacity to process product mass and volume. According to various sources [1] the shift from concerns over manufacturing capacity to concerns over costs has been occurring over the past few years.

The problems associated with recombinant protein expression are that the vast body of data is associated with one product type (antibodies) and with one cell type (mammalian cells). This system has high production costs associated with not only the capital-intensive infrastructure but also the purification costs. Other expression systems include yeast (GenWay Biotech), insect cell culture (Baculovirus Expression Vector System (BEVS), developed by Max Summers, Gale Smith and colleagues in Texas A&M Univeristy), whole insect systems (Chesapeake Perl), plant based systems (Greenovation) or any of the bacterial expression systems. All of these have their individual benefits and drawbacks but unless they use a tag-based purification strategy they will suffer the same as for native conformation expression in mammalian cells. The cost of purification will account for very high percentages of the final cost of goods. In the case of using a tag-based purification process they suffer from the need to remove the tag.

The search for inexpensive production systems capable of producing large quantities of recombinant protein has resulted in the development of new technology platforms, for example, the accumulation of proteins in the endoplasmic reticulum (ER) based on the delivery there by the KDEL (Lys-Asp-Glu-Leu) peptide [3]. This allows for the accumulation of protein in the ER of the host but does not allow for production of the protein in a native conformation as the KDEL sequence remains attached. Other systems include the Elastin-like polypeptides (ELP) [4], based on repeats of the amino acids "VPGXG" that undergo a temperature dependant phase transition and have utility in the purification of recombinant proteins but can also enhance the expression of recombinant fusion proteins. Again this tag is retained by the mature protein and unlike the KDEL sequence the ELP does not accumulate the protein in any particular location. Another protein accumulation technology is the Stratosome Biological Systems technology from Sembiosys. The Stratosome technology is based on the accumulation of the recombinant protein fused to a targeting tag (targeted to oil bodies) called Oleosin, this tag accumulates the protein in oil bodies with the protein expressed on the surface of the structure. The protein can accumulate to up to 5% of the total seed protein [5] in the safflower host. This system accumulates the protein in a defined place and uses this localised structure to aid in the purification process of the protein, but the protein of interest must also be cleaved from not only the oleosin tag but the structure to which it is attached.

The use of tags to aid in protein expression serves a two-fold purpose; firstly, as in the case of the KDEL, to locate the protein in a particular area, or, secondly, as in the case of the ELP, to aid in purification. There are other tags which fulfil both these purposes. The Glutathione S-transferase (GST) tag, for example, aids in increasing solubility of some proteins which are deemed difficult to express, but the same tag can hinder the folding of the very same protein resulting in a non-native conformation. This taken in conjunction with the fact that the GST-tag then will have to be removed by enzymatic means to free the fusion product. The same can be said for the other tags for purification, e.g., the His-tag is very small at about 0.85 kDa when compared to the GST-tag at about 27 kDa, but the principle is the same. Since these tags can alter the biological activity of target proteins and interfere with protein crystallization studies, many biological and biomedical applications require that the tag be removed from the target protein. Most commonly used methods involve the addition of exogenous site-specific proteases to cleave the affinity tag off the target protein at engineered sites [6]. Unfortunately, high levels of endoprotease must often be applied for extended periods of time, and this can result in undesirable cleavages within the target protein. Furthermore, these endoproteases are costly, often exhibit poor solubility, and require the inclusion of additional chromatography steps to remove the exogenous protease [7].

Although these tag-removal systems alleviate problems associated with the presence of the tag in the final purified protein, they have several principal drawbacks: 1) reduced stability of the target protein; 2) extended length of purification protocols due to additional cleavage and protease-removal steps, which may hamper high-throughput purification approaches and result in loss of target protein; and 3) the nature of protease cleavage mechanisms often result in generation of protein products that contain extra residues on their N-termini and therefore a non-native conformation.

Over the past few decades the production of recombinant proteins for use in various fields including therapeutic, industrial, and cosmetic or as nutraceuticals has had some great success. The exploitation of different prokaryotic and eukaryotic hosts has been shown to produce polypeptide products for such applications. However, high costs associated with low expression levels of even higher recovery and purification processes can nullify these positive gains, though current research is ongoing to improve aspects in both expression and recovery. The improvements in the expression levels associated with some products of interest has left gaping holes and bottlenecks in the purification of these same products, and according to many the bottleneck in pharmaceutical production has moved to the purification stage and requires improvements and according to some authors [8], the purification is the most costly part in the production of a biomolecule, with 50-80% of total costs incurring at the purification stage. So it is not only the lack of expression or low levels of expression which are holding back development of certain biomolecules but also the high purification costs which are associated.

In order to address these issues it is essential to develop a new approach to the expression and purification issues. The novel technology here described will address both of these issues, increasing the levels of expression by targeting and accumulating the proteins inside the mitochondria of a host cell or host system thereby reducing the degradation which occurs with some expression systems, e.g. proteolysis is a problem of the BEVS system due to its lytic nature, and can, therefore, be a critical issue [9]. This development will concomitantly allow for a reduction in the steps for the purification of the product of interest, the target for the initial purification being common to all the targets; the mitochondria, which will also be independent from the host of choice as the mitochondria is common to all eukaryotes. The purification of mitochondria is a commonly used process for those wishing to study the metabolic status of mitochondria, the biochemical, physiological and molecular applications [10], and there exist a number of protocols and kits for the purification of mitochondria (e.g., Qiagen's Qproteome Mitochondria Isolation Kit, Product Number 37612, or the Pierce Mitochondria Isolation Kit for Cultured Cells, Product Number 89874). These kits rely upon the same basic principle i.e. cell lysis and differential centrifugation to isolate or enrich the mitochondrial fraction. The mitochondria can be isolated from the cell mass and therefore any protein contained therein, but the currently available technologies do not take advantage of this aspect either for expression or accumulation and subsequent enrichment. There are a number of examples of technologies employing naturally occurring mitochondrial targeting sequences.

The Organelle Lights product from Invitrogen, Cat. No. 036229 [11] contains the red fluorescent protein (RFP) sequence fused to the presequences from E1alpha pyruvate dehydrogenase (Table 1), which according to currently available software [MitoProtII (http://ihg2.helmholtz-muenchen.de/ihg/mitoprot.html)] [12] will not leave the RFP in a native conformation. There is also the pTagRFP-mito from Evrogen, Cat. No. FP147 [13] which contains the same RFP sequence but has the presequences from the subunit VIII of human cytochrome C oxidase fused to the N-terminal (Table 1), which again does not leave a native conformation after analysis by MitoProtII. Indeed the manufacturers do not claim that it will do, they use the RFP to label an organelle and permit its visualisation upon excitation by an adequate energy source. They do not express and accumulate a protein there for any other purposes nor do they indicate or mention any cleavage of the mitochondrial targeting sequence. The Invitrogen Organelle Lights product is for "The visualization of specific parts of living cells using fluorescence microscopy, flow cytometry, or high content imaging" and the Evrogen product pTagRFP-mito is "intended for red (orange) fluorescent labelling of mitochondria in living cells". The mitochondrial targeting sequences employed are to send a visualisation signal to the mitochondria and nothing more. When the signals are analysed with the fusion partner the cleavage is incorrect with the E1alpha pyruvate dehydrogenase sequence not being recognised as a cleavage sequence and instead removing the first 2 amino acids of the fusion partner, (in the case of the Invitrogen product), and with the subunit VIII of human cytochrome C oxidase being cleaved before the end of the signal sequence therefore leaving 3 amino acids fused to the target protein (in the case of the Evrogen product), as shown in Table 1. In both cases the fusion partner is the same, i.e. a red fluorescent protein with the first 10 amino acids being VSKGEELIKE (Invitrogen Cat. No. 036229 and Evrogen Cat. No. FP147).

Table 1

| MitoProtII analysis of commercial sequences | | |
|---|---|---|
| **Targeting Sequence** | **Fusion Sequence** | **Cleavage Product** |
| (Invitrogen)<br>MRKMLAAVSRVLSGASQKPASRVLVASRN | VSKGEELIKE | MRKMLAAVSRVLSGASQKPASRVLVASRNVS |
| (Evrogen)<br>MSVLTPLLLRGLTGSARRLPVPRAKIHSL | VSKGEELIKE | MSVLTPLLLRGLTGSARRLPVPRAKI |

These sequences are naturally occurring and relatively large for protein expression (3.2 kDa when compared to the 0.8 kDa of the hexahistidine tag for example) especially if the tag cannot be used for purification purposes or leaves the fusion protein in a non-native conformation. In fact according to the MitoProtII analysis the possibility of the products being directed to the mitochondria is 58% for the E1alpha pyruvate dehydrogenase and 29% for the subunit VIII of human cytochrome C oxidase. The E1alpha pyruvate dehydrogenase and the subunit VIII of human cytochrome C oxidase were analysed with 10 different proteins and the results are shown in Table 2.

Table 2

| MitoProtII Analysis on various proteins | |
|---|---|
| **Protein** | **N-Terminal Amino Acid Sequence** |
| Nerve Growth Factor Receptor | SILFYVIFL |
| Gastric Lipase | WLLLTMASL |
| Epidermal Growth Factor Receptor | NSDSECPLS |
| Alpha Amylase | AKHSTTMSC |
| Hepatitis-B Surface Antigen | GQNLSTSNP |
| Influenza hemagglutinin | EKIVLLLAI |
| Tumor Necrosis Factor Receptor | RAQRSLERRR |
| cGMP-Gated Ion Channel Protein | KNNIINTQQ |
| Lysophosphatidic Acid Receptor 3 | NECHYDKHM |
| Epidermal Growth Factor Receptor | LLFLILLLP |

The end result was the same as for the red fluorescent protein, i.e. in each of the proteins shown in Table 2 the E1alpha pyruvate dehydrogenase removed the first 2 amino acids of each of the target proteins, and in the case of the subunit VIII of human cytochrome C oxidase it left 3 amino acids from the targeting sequence attached to the 10 proteins. These results were generated by the inventors using the sequences indicated and the MitoProtII software to analyse the positions of cleavage. For labelling an organelle with a fluorescent protein this may not be too damaging, but in the case of a potential biopharmaceutical like those shown in Table 2, it could mean the difference between success and failure. This problem of unspecific cleavage, leaving behind or adding amino acids to the target sequence coupled to the fact that the sequences are natural and specific for a single protein leaving a non-native sequence when fused to other proteins is demonstrated in Table 2. In fact there exist a few mitochondrial proteins which contain a mitochondrial targeting sequence which is not cleaved off, these include the chaperonin 10 (Cpn10) among the mitochondrial matrix proteins synthesized without a cleavable targeting signal [14].

Therefore, there exists the need of providing more efficient systems for producing peptides and proteins in eukaryotic host systems which solve total or partially the above mentioned drawbacks, mainly those related to the expression and purification of the recombinant peptides and proteins and the native conformation issues.

**Brief Summary of Invention**

The problem to be solved by the present invention is to provide an alternate and more efficient system for producing peptides and proteins of interest in a eukaryotic host system. In order to solve said problem, particularly, to address both the issue of improving on the expression and purification of recombinant peptides and proteins and the native conformation issue, a novel technology based on the expression of a protein fusion comprising a synthetic mitochondrial targeting sequence (MTS) and a product (protein or peptide) of interest has been developed to increase gains in expression and accumulation levels and to simplify the recovery and purification processes in eukaryotic host cells and organisms. As for most but not all of the native mitochondrial targeting sequences, the newly designed MTS provided by the instant invention are capable of sending a protein to the mitochondria where it accumulates and at the same time has the self same tag removed as it enters the mitochondrial matrix due to the presence in the fusion protein of an amino acid sequence that is specifically cleavable by a mitochondrial protease enzyme. Illustrative non-limitative examples of some classes of proteins which have proven very difficult to express in quantities large enough to feed the pipeline of therapeutic proteins such as GPCRs, kinases, ion channels, blood plasma proteins, vaccines and antibodies can efficiently be produced in accordance with the teachings of the instant invention.

Thus, the invention contemplates the production of recombinant peptides and proteins (polypeptides) in eukaryotic cells and organisms as host systems after transformation of the host with an appropriate vector. Notably, a target polypeptide is fused to a synthetic MTS and expressed as a fusion product, said fusion product being driven to the mitochondria mediated by said MTS. There the fusion protein is broken to release the product of interest and the MTS; the MTS is specifically removed enzymatically by mitochondrial processing enzymes. The target product of interest is then stored inside the mitochondria and accumulates in the host cell or organism. The isolation and accumulation within the mitochondria allows for protection for the product of interest from the cellular environment, and also for protection of the cell from the product of interest (in the case of a toxic product for example).

The herein solution is based on the ability of an optimised short artificially designed MTS not only to transport (deliver) peptides/proteins to the mitochondria of the host system but to be effectively and efficiently removed once internalised. Further, the designed MTS provided by this invention confers not only the ability to transport a product of interest in the form of a fusion protein to the mitochondria wherein said MTS is efficiently removed once internalised to render the product of interest but at the same time allows the accumulation within a double membrane system (i.e., mitochondria), effectively providing a safe-lock system where the protein is protected from the cellular environment and the cellular environment from the protein. Thus, the mitochondria provide a storage and accumulation environment where, due to the internal chaperones native to the system, the product of interest (protein, peptide or polypeptide) is correctly folded and bioactive. Accumulating within the mitochondria has numerous advantages, such as, for example:

1. The folding of proteins is energy dependent and where better to find the energy to perform the folding than at the source.
2. Folding requires chaperones and the mitochondria are replete with them.
3. The mitochondria is used to dealing with the import/folding process as the vast majority of its proteins are imported and treated in the way.
4. According to current work by Zhao et al. [15] the accumulation of unfolded protein within the mitochondrial matrix results in the transcriptional upregulation of nuclear genes encoding mitochondrial stress proteins such as chaperonin 60 and chaperonin 10. So that even in the case of there being unfolded proteins present, the mitochondria respond by producing more chaperones to aid in the folding process.
5. Mitochondria are semi-autonomous in that they have the ability to self-replicate; therefore their numbers are not limited to the cell cycle of the host cell.
6. Mitochondria have a natural processing system for the removal of the MTS's so no costly enzymatic cleavage system is needed to produce the protein of interest in a native format.
7. It is axiomatic to assume that as mitochondria are present within all eukaryotes and having a similar if not exact origin, that therefore the process of protein import, cleavage, folding and accumulation would also be similar/exact.

The use of a MTS fusion protein based-system to accumulate, cleave and fold a product of interest in a host system constitutes a novel and efficient approach to protein expression within a eukaryotic host system.

The Example illustrates the invention where the MTS fusion protein based-system to accumulate a recombinant fluorescent protein (RFP) is described. Various MTS domains were engineered, identified as MIS-VII (SEQ ID NO: 2), MIS-XIII (SEQ ID NO: 3) and MIS-XIV (SEQ ID NO: 4), to serve as fusion partners through computer generated software and were designed to contain not only an MTS which is capable to deliver the protein to the mitochondria, but also to be processed correctly and to be removed by internal mitochondrial proteases. Mature RFP coding region was fused at the C-terminus of the MTS domains and expressed in yeast (*Kluyveromyces lactis*) cells. Mitochondria were specifically stained with the MitoTracker Green (MTG) fluorescent stain and the RFP proteins were accumulated inside the mitochondrion of the transformed yeast cells as observed by confocal fluorescent microscopy. The MTS sequence with highest proportion of RFP within the mitochondria (after images from the MTG (Invitrogen, Cat. No. M-7514) and RFP fluorescent images were over-laid) were defined as the mitochondrial targeting sequences (MTS), having the greatest ability to drive the expression and accumulation of the RFP to the mitochondria. The sequence integrity of the RFP was confirmed in the example by MALDI-TOF-TOF analysis, leaving the mature RFP in a native conformation. The example confirms the delivery to the mitochondria, accumulation in the same organelle and subsequent cleavage of the designed MTS to leave the target protein in a mature and intact conformation (correct folding).

Accordingly, an aspect of this invention relates to a nucleic acid comprising: a first polynucleotide containing a nucleotide sequence that encodes a MTS, wherein said MTS comprises the amino acid sequence shown in SEQ ID NO: 1 (RRRA), and is capable of directing the protein towards a mitochondria; a second polynucleotide containing a nucleotide sequence that encodes an amino acid sequence that is specifically cleavable by a mitochondrial protease enzyme; and a third polynucleotide containing the nucleotide sequence that encodes a product of interest; wherein the 3' end of said first polynucleotide is linked to the 5' end of said second polynucleotide and the 3' end of said second polynucleotide is linked to the 5' end of said third polynucleotide.

In another aspect, this invention relates to a nucleic acid construct comprising said nucleic acid.

In a further aspect, the invention relates to a vector containing said nucleic acid or nucleic acid construct.

In a further aspect, the invention relates to a cell having said nucleic acid, nucleic acid construct or vector.

In a further aspect, the invention relates to a transformed host system having said nucleic acid, nucleic acid construct or vector.

In a further aspect, the invention relates to a transgenic host system (whole/entire organism for example a plant or an animal wherein genetic material has been incorporated from an external source via genetic engineering) comprising, integrated in its genome, said nucleic acid.

In a further aspect, the invention relates to a method for producing a product of interest in a host system based on the MTS mitochondrial targeting technology wherein said MTS comprises the amino acid sequence shown in SEQ ID NO: 1, and is capable of directing the protein towards a mitochondria.

In a further aspect, the invention relates to a fusion protein, said fusion protein having an amino acid sequence corresponding to the above mentioned nucleic acid.

In a further aspect, the invention relates to a method for delivering a product of interest to a mitochondria, comprising the steps of:

a) preparing a nucleic acid construct of the invention;
b) introducing said nucleic acid construct of the invention into an eukaryotic cell to produce a transformed cell; and
c) expressing the nucleic acid construct of the invention from the nucleus of the transformed cell.

In a further aspect, the invention relates to a pharmaceutical composition comprising the above mentioned nucleic acid, or nucleic acid construct, or vector, or fusion protein, together with a pharmaceutically acceptable vehicle.

In a further aspect, the invention relates to a composition of matter selected from the group consisting of the above mentioned nucleic acid, nucleic acid construct, vector or fusion protein, for use in the treatment of a mitochondrial disorder in a subject. In a particular embodiment, subject is an eukaryotic organism, including human beings. The method for treating a mitochondrial disorder in a subject in need thereof based on the use of said composition of matter, constitutes a further aspect of this invention.

In a further aspect, the invention relates to a mitochondria comprising the above mentioned fusion protein or a product of interest resulting from the enzymatic cleavage into the mitochondria of said fusion protein.

In a further aspect, the invention relates to a peptide comprising the amino acid sequence shown in SEQ ID NO: 1. The peptide is capable of directing the protein towards a mitochondria. In a particular embodiment, said peptide is a peptide of formula (I) as defined below. In another particular embodiment, said peptide is a peptide whose amino acid sequence is shown in SEQ ID NO: 2, SED ID NO: 3 or SEQ ID NO: 4.

**Brief Description of the Drawings**

Figure 1 schematically shows the vectors of the invention identified as pMIS-VII-RFP, pMIS-XIII-RFP and pMIS-XIV-RFP (Example 1). Each vector contains the corresponding mitochondria targeting sequence (MTS) and the red fluorescent protein (RFP) as a fusion product [(MIS-VII)-RFP, (MIS-XIII)-RFP and (MIS-XIV)-RFP, respectively].

Figure 2 schematically shows the control positive plasmid identified as pPOS-CONTROL (Example 1). The pPOS-CONTROL contains the control sequence of cytochrome-C-oxidase and the red fluorescent protein as a fusion product.

Figure 3 schematically shows the control negative plasmid identified as pNEG-CONTROL (Example 1). The pNEG-CONTROL contains an alpha-mating factor for secretion of the red fluorescent protein as a fusion product.

Figure 4 shows the results of the co-localisation images of the ability of the MTS to deliver the protein to mitochondria. The MTS in the images are the leader sequence for the cytochrome-C-oxidase and the MTS of the current invention. Briefly, this figure shows the subcellular localisation of fusion proteins (MIS-VII)-RFP, (MIS-XIII)-RFP and (MIS-XIV)-RFP in transgenic yeast cells: (left) immunolocalisation of (MIS-VII)-RFP, (MIS-XIII)-RFP and (MIS-XIV)-RFP in transgenic lines using the innate fluorescence of the fluorescent protein excited by the corresponding laser (at 543nm); (middle) immunolocalisation of mitochondrion using MitoTracker green dye for fluorescent labelling of mitochondria using the laser (at 488nm) to excite and detect the fluorescence generated; and (right) the images from left and middle superimposed to demonstrate that the RFP protein is efficiently targeted to mitochondrion.

Figure 5 shows the results of the Manders co-localisation analysis. Manders overlap coefficient indicates an overlap of the signals and thus represents the true degree of colocalization.

## Detailed Description of the Invention

In an aspect, the invention provides a nucleic acid, hereafter referred to as the nucleic acid of the invention, comprising:

- a first polynucleotide containing a nucleotide sequence that encodes a mitochondria-targeting sequence (MTS), wherein said MTS comprises the amino acid sequence shown in SEQ ID NO: 1 (RRRA), and is capable of directing the protein towards a mitochondria;
- a second polynucleotide containing a nucleotide sequence that encodes an amino acid sequence that is specifically cleavable by a mitochondrial protease enzyme; and
- a third polynucleotide containing the nucleotide sequence that encodes a product of interest;

wherein the 3' end of said first polynucleotide is linked to the 5' end of said second polynucleotide and the 3' end of said second polynucleotide is linked to the 5' end of said third polynucleotide.

The first polynucleotide contains the nucleotide sequence that encodes the MTS, said MTS comprises the amino acid sequence shown in SEQ ID NO: 1, and is capable of directing the protein towards mitochondria. The SEQ ID NO: 1 (RRRA) constitutes the Core Domain Element (CDE) and is comprised of Arginine (R) [an electrically charged (positive) amino acid] and Alanine (A) [a non-polar hydrophobic amino acid]. The central CDE, which is present in all the MTS according to the instant invention, has been demonstrated capable of forming a helical structure, the helical structure which aids in the process of transport to the mitochondria. Analysis by NetSurfP (http://www.cbs.dtu.dk/services/NetSurfP/) of said sequence demonstrated that helical nature of the sequence, the results of which are shown in Table 3 (Example 1).

However, other proteins containing a core sequence comprising R (Arg) and A (Ala), quite similar to the CDE according to the invention, e.g., the core sequence RRA, are unable to direct the protein towards a mitochondria. Illustrative, non-limitative, examples of said proteins containing said core sequence RRA include the hypothetical protein AZL_c03820 [*Azospirillum* sp. B510] with an NCBI Reference Sequence: YP_003452219.1, which contains the sequence RRARRARR at positions 7 to 14 of said protein and is not directed to the mitochondria according to analysis by MitoProtII, and the cellulose synthase subunit B [*Azospirillum* sp. B510] with an NCBI Reference Sequence: YP_003452353.1, which contains the RRAR sequence at positions 12 to 15 of said protein and again is not capable of being directed to the mitochondria according to analysis by MitoProtII. According to analysis by the inventors using the MitoProtII software, neither of the aforementioned proteins (AZL_c03820" y "cellulose synthase subunit B) although having a sequence similar to the sequences of this invention have the capabilities to be directed to the mitochondria.

The MTS according to the invention contains, at least, one CDE sequence [SEQ ID NO: 1]. Thus, in a particular embodiment, said MTS comprises one CDE sequence. In another particular embodiment, said MTS comprises more than one CDE sequences, for example, 2, 3, 4, 5, or even more CDE sequences.

In a particular embodiment, the MTS is a peptide of formula (I)

$$(X1)_n\text{-RRRA-}(X2)_m$$

(I)

wherein

X1 is RL, RAA or RA, wherein A is alanine, L is leucine, and R is arginine;

X2 is ARSLARSAARR, ARR or RR, wherein A is alanine, L is leucine, and R is arginine are those previously defined, and S is serine; and

m and n are, independently, 0 or 1.

In another particular embodiment, the MTS is a peptide whose amino acid sequence is shown in SEQ ID NO: 2 (identified as MIS-VII in Example 1), SEQ ID NO: 3 (identified as MIS-XIII in Example 1) or SEQ ID NO: 4 (identified as MIS-XIV in Example 1).

Since the MTS according to the invention may contain one or more CDE sequences, in a particular embodiment, the first polynucleotide comprises at least once the nucleotide sequence shown in SEQ ID NO: 5, whereas in another particular embodiment, the first polynucleotide comprises two or more times the nucleotide sequence shown in SEQ ID NO: 5, e.g., 2, 3, 4, 5 or even more.

Thus, in a particular embodiment, the first polynucleotide comprises the nucleotide sequence shown in SEQ ID NO: 5 which encodes SEQ ID NO: 1.

In another particular embodiment, the first polynucleotide comprises the nucleotide sequence shown in:

- SEQ ID NO: 6, which encodes the peptide whose amino acid sequence is shown in SEQ ID NO: 2 (MIS-VII), or
- the nucleotide sequence shown in SEQ ID NO: 7, which encodes the peptide whose amino acid sequence is shown in SEQ ID NO: 3 (MIS-XIII), or
- the nucleotide sequence shown in SEQ ID NO: 8, which encodes the peptide whose amino acid sequence is shown in SEQ ID NO: 4 (MIS-XIV)].

The <u>second polynucleotide</u> contains a nucleotide sequence that encodes an amino acid sequence that is specifically cleavable by a mitochondrial protease enzyme. Although any amino acid sequence that is specifically cleavable by a mitochondrial protease enzyme can be present in the fusion protein encoded by the nucleic acid of the invention, in a particular embodiment, the second polynucleotide comprises a nucleotide sequence that encodes a protease cleavage site, for example, an amino acid sequence site which is cleavable by a protease such as the mitochondrial processing protease (MPP). MPP is a general peptidase, acting on hundreds of mitochondrial precursor sequences, recognizing a distinct cleavage site and specifically removing the pre-sequence. The mechanism of MPP recognition of the cleavage site on a precursor has yet to be clarified. The importance of arginine residues proximal and distal to the cleavage site has been shown experimentally by site-directed mutagenesis or with synthetic presequence peptides [16, 17 and 18]. Presequences do not share sequence similarity and no consensus for processing has been found, except that many mitochondrial precursors contain a loosely conserved motif with an arginine residue at the -2 or -3 position upstream of the cleavage site [19 and 20]. The importance of these arginines for presequence recognition by MPP has been shown by experimental studies [17 and 21]. An example of a protein which contains a cleavable pre-sequence is the E1alpha pyruvate dehydrogenase, which according to Invitrogen (Cat. No. 036229), has a cleavage site of MRKMLAAVSRVLSGASQKPASRVLVASRN (SEQ ID NO: 16).

In a particular embodiment, the second polynucleotide can be concomitantly contained in the first polynucleotide, i.e., said first polynucleotide may contain, at its 3' end, a nucleotide sequence that encodes an amino acid sequence that is specifically cleavable by enzymatic means, such as, by a mitochondrial protease enzyme (i.e., the second polynucleotide).

The <u>third polynucleotide</u> contains the nucleotide sequence that encodes a product of interest. This invention does not discriminate in the expression of the product of interest, the product of interest, in a preferred embodiment, being proteinaceous in nature (e.g., protein or peptide), e.g., hormones, cytokines, receptors, enzymes or other active (bio)molecules.

The terms "protein" and "peptide", as used herein, refer in general to a chain of amino acids linked by peptide bonds. Unless otherwise indicated, the term "protein" includes protein domains, including any post-translational modification(s). Similarly, unless otherwise indicated, the term "peptide" includes polypeptide. One of skill in the art, upon reading the instant specification, will appreciate that these terms also include structural analogs and derivatives, e.g., proteins or peptides having conservative amino acid insertions, deletions, or substitutions, peptidomimetics, and the like. Said terms "protein" and "peptide" can overlap in meaning and can be used interchangeably. "Protein" is generally used to refer to the complete biological molecule in a stable conformation; and "peptide" is generally reserved for any single linear chain of amino acids, usually regardless of length, often lacking a stable three-dimensional structure.

The nature and application of said products of interest being independent to the production system herein described and dependent upon the nature of the product of interest (protein/peptide). The "product of interest" may be expressed from a naturally occurring gene, a mutated gene or a synthetic gene. Virtually, any product of interest may be expressed by the system provided by the instant invention; nevertheless, in a preferred embodiment, the product of interest will have effect in treating human or animal conditions, be applicable in *in-vitro* or *in-vivo* kits, industrial, environmental and chemical applications. These may include but not be limited to a G protein-coupled receptor (GPCR), a membrane protein used in therapeutic and/or diagnostic assays, such as, for example, an acetylcholine receptor, an hydroxytryptamine receptor, etc., and enzymes for industrial use, for example, lipases and cellulases used in biofuel production, etc.

According to the invention, the 3' end of said first polynucleotide is linked to the 5' end of said second polynucleotide and the 3' end of said second polynucleotide is linked to the 5' end of said third polynucleotide; in a particular and preferred embodiment, said first, second and third polynucleotides are in reading frame.

The nucleic acid of the invention may be DNA or RNA (e.g., mitochondrial DNA or RNA), including synthetic forms and mixed polymers, and both sense and antisense strands. Nucleic acids for use in the present invention may be isolated from cell cultures using known methods or may be obtained by using conventional techniques known for the skilled person in the art. Additionally, the nucleic acid sequences may be prepared by a variety of techniques known to those skilled in the art, including, without limitation, the following: restriction enzyme digestion of nucleic acid; ligating the different polynucleotides constituting the nucleic acid of the invention; and automated synthesis of oligonucleotides, using commercially-available oligonucleotide synthesizers, such as the Applied Biosystems Model 392 DNA/RNA synthesizer. A review of said conventional techniques may be found, for example, in "Molecular cloning, a Laboratory Manual", 2nd ed., by Sambrook et al., Cold Spring Harbor Laboratory Press, 1989. The construction of some vectors containing a nucleic acid of the invention is disclosed in Example 1 and illustrated in Figure 1.

The nucleic acid of the invention encodes a fusion protein comprising, from the N-terminal end to the C-terminal end, a MTS, wherein said MTS comprises the amino acid sequence shown in SEQ ID NO: 1 (RRRA), and is capable of directing the protein towards a mitochondria, an amino acid sequence that is specifically cleavable by a mitochondrial protease enzyme, and a product of interest.

Therefore, in a further aspect, the invention provides a fusion protein, hereinafter referred to as the fusion protein (or product) of the invention, comprising:

- an amino acid sequence comprising a mitochondria-targeting sequence (MTS), wherein said MTS comprises the amino acid sequence shown in SEQ ID NO: 1 [RRRA], and is capable of directing a protein towards a mitochondria;
- an amino acid sequence that is specifically cleavable by a mitochondrial protease enzyme; and
- a product of interest;

said fusion protein being the expression product of the nucleic acid of the invention.

The fusion protein of the invention is delivered to the mitochondria wherein it is cleaved to render the product of interest inside the mitochondria, in the eukaryotic host system or cell, wherein it is accumulated. The enzymatically cleavable site, which is present at the C-terminus of the MTS allows to recover the product of interest afterwards. The product of interest may be then isolated and purified by conventional means, for example, by lysing the cells and subjecting the mass to isolation or enrichment of the mitochondrial fraction; the mitochondria can be isolated from the cell mass and therefore any protein contained therein; there exist a number of protocols and kits for the purification of mitochondria (e.g., Qiagen's Qproteome Mitochondria Isolation Kit, Product Number 37612, or the Pierce Mitochondria Isolation Kit for Cultured Cells, Product Number 89874, etc.). Therefore, the fusion protein of the invention constitutes a novel and successful approach to accumulate a product of interest.

Thus, the use of a MTS fusion protein based-system to accumulate, cleave and fold a product of interest in a host system constitutes a novel and efficient approach to protein expression within a eukaryotic host system. In fact, the novel technology herein described allows for an increase in the levels of expression of a product of interest (e.g., a protein) by targeting and accumulating the protein inside the mitochondria of a host cell or host system thereby reducing the degradation which occurs with some expression systems, e.g. BEVS system, and, concomitantly allows for a reduction in the steps for the purification of the product of interest since it is accumulated in mitochondria.

In a particular embodiment, the fusion protein of the invention is the expression product of the nucleic acid of the invention in a host system, e.g., *K. lactis* (Example 1), wherein in this specific but non-limiting example, the host system is independent having requirements of a mitochondria to function correctly.

As shown in Example 1, the protein of interest of the invention is accumulated in mitochondrion within a host system, the expression of the protein of interest having little or no effect on the growth of the host system.

The product of interest may be then pre-enriched and purified by conventional means, as mentioned above, thereby the invention presented constitutes a novel approach to accumulate a product of interest with its native/natural sequence even if the localisation of the protein product in the mitochondria is not habitual for the product in question.

In another aspect, the invention relates to a nucleic acid construct, hereinafter referred to as the nucleic acid construct of the invention, comprising (i) the nucleic acid of the invention, and (ii) a transcription regulatory element that regulates the transcription of the nucleic acid of the invention (i), said transcription regulatory element (ii) being functional in eukaryotic systems or cells. Said nucleic acids (i) and (ii) are operatively linked.

Practically any eukaryotic functional transcription regulatory element may be used. In an embodiment, said transcription regulatory element (ii) is, preferably, tissue-specific, i.e., it can regulate the transcription of the nucleic acid of the invention in a specific tissue. The transcription regulatory element (ii) may comprise a promoter functional in eukaryotes. The transcription regulatory element (ii) may also contain a transcription termination sequence and may also contain a translation enhancer functional in eukaryotes.

In a particular embodiment, said eukaryotic system or cell is a host cell or a transgenic host system, and said transcription regulatory element (ii) is specific for said host cell or transgenic host system; in a further particular embodiment, said transcription regulatory element (ii) comprises a functional promoter, a transcription enhancing sequence, a transcription termination sequence and a translation enhancer functional in said host cell or transgenic host system.

The nucleic acid sequence or the construct provided by this invention may be inserted into an appropriate vector. Therefore, in a further aspect, the invention provides a vector, hereinafter referred to as the vector of the invention, comprising the nucleic acid of the invention or a nucleic acid construct of the instant invention. The choice of the vector (which may include plasmids, cosmids or viral vectors) may depend on the host cell wherein it is to be subsequently introduced and is suitable for transformation of eukaryotic hosts either in an epigenetic format or is integrated into the genome of said host cell and is replicated along with the chromosome (or chromosomes) in which it has been integrated. The application of techniques found in Molecular cloning, a Laboratory Manual", 2nd ed., by Sambrook et al., Cold Spring Harbor Laboratory Press, 1989, may lead to the generation of this and other vectors.

In a further aspect, the invention provides a transformed host system, the said transformed host system having been transformed by conventional methods with the nucleic acid of the invention, or with a construct of the invention or with a vector of the invention. As used herein, the term "transformed host system" includes all eukaryotic cells/systems/hosts which include but is not limited to the following kingdoms: animalia, plantae, fungi, amoebozoa, chromalveolata, rhizaria and excavate and also refers to the progeny of the aforementioned transformed host systems which contain the nucleic acid of the invention. The term "transformed host system" refers to eukaryotic cells including cells of animal, plants, fungi and protists, and eukaryotic viruses such as retrovirus, adenovirus, baculovirus and the transformation of the same [22].

In a further aspect, the invention provides a transgenic host system, engineered to contain a novel, laboratory designed transgene, said transgenic host system comprising, integrated in its genome, the nucleic acid of the invention. Said transgenic host system may be obtained by means of conventional techniques, for example, through the use of conventional antisense mRNA techniques and/or overexpression (in sense silencing) or others, for example, by using binary vectors or other vectors available for the different transformation techniques currently in use. In a particular embodiment, said transgenic host system is a transgenic plant host system. Examples of transgenic plant host systems provided by the present invention include both monocotyledon and dicotyledonous plants, and, specifically, cereals, legumes, cruciferous, solanaceous, etc.

A transgenic organism of the invention is preferably a multicellular eukaryotic organism, such as an animal, a plant, a fungus, etc. Animals include animals of the phyla cnidaria, ctenophora, platyhelminthes, nematoda, annelida, mollusca, chelicerata, uniramia, crustacean and chordata. Chordates includes vertebrate groups such as mammals, birds, reptiles and amphibians. Particular examples of mammals include non-human primates, cats, dogs, ungulates such as cows, goats, pigs, sheep and horses and rodents such as mice, rats, gerbils and hamsters. Plants include the seed-bearing plants angiosperms and conifers. Angiosperms include dicotyledons and monocotyledons. Examples of dicotyledonous plants include tobacco (*Nicotiana plumbaginifolia* and *Nicotiana tabacum*), arabidopsis (*Arabidopsis thaliana*), *Brassica napus, Brassica nigra, Datura innoxia, Vicia narbonensis, Vicia faba,* pea (*Pisum sativum*), cauliflower, carnation and lentil (*Lens culinaris*). Examples of monocotyledonous plants include cereals such as wheat, barley, oats and maize.

Techniques for producing transgenic animals are well known in the art. A useful general textbook on this subject is Houdehine, Transgenic animals - Generation and Use (Harwood Academic, 1997) - an extensive review of the techniques used to generate transgenic animals from fish to mice and cows.

Techniques for producing transgenic plants are also well known in the art. Typically, either whole plants, cells or protoplasts may be transformed with a suitable nucleic acid construct of the invention encoding a product of interest. There are many methods for introducing transforming DNA constructs into cells, but not all are suitable for delivering DNA to plant cells. Suitable methods include *Agrobacterium* infection (see, among others, Turpen et al, 1993, J. Virol. Methods, 42: 227-239) or direct delivery of DNA such as, for example, by PEG-mediated transformation, by electroporation or by acceleration of DNA coated particles. Acceleration methods are generally preferred and include, for example, microprojectile bombardment. A typical protocol for producing transgenic plants (in particular moncotyledons) is disclosed in U.S. Patent No. 5,874,265.

The nucleic acid of the invention is useful for producing a product of interest in a transformed host system or in a transgenic host system. Therefore, in a further aspect, the invention provides a method for producing a product of interest in a transformed host system or in a transgenic host system, which comprises growing a transformed or transgenic host system provided by the invention, under conditions that allow the production and expression of said product of interest in the form of a fusion protein. The fusion protein is transported to the mitochondria where it accumulates and is folded into its native configuration via the mitochondrial refolding mechanism (including chaperones). The MTS is processed by the mitochondrial processing machinery and is specifically cleaved, leaving the product of interest intact and complete in its native configuration. The product of interest can be enriched, isolated and purified by known conventional techniques according to the specific characteristics of the individual product of interest. Therefore, the method provided by the invention further comprises, if desired, the isolation and purification of said fusion protein, and, the release of said product of interest from said fusion protein.

In a particular embodiment, said transformed or transgenic host system is a transformed or transgenic plant host system.

Therefore, the invention provides a system to direct and accumulate recombinant products of interest in host system mitochondria.

Thus, in another aspect, the invention provides a method for delivering a product of interest to a mitochondria, comprising the steps of:

a) preparing a nucleic acid construct of the invention;
b) introducing said nucleic acid construct of the invention into an eukaryotic cell to produce a transformed cell; and
c) expressing the nucleic acid construct of the invention from the nucleus of the transformed cell.

The nucleic acid construct of the invention is expressed to produce the product of interest in the form of an enzymatically cleavable fusion product in a mitochondria and said fusion protein enters the mitochondria.

The particulars of the nucleic acid construct of the invention have been previously mentioned. In a particular embodiment, the nucleic acid construct of the invention may be prepared by methods known in the art, including those described below. Vectors, promoters, and ribosomal entry sites, including those disclosed herein, may be used, in conjunction with standard techniques, to prepare the nucleic acid construct of the invention. Vectors that may be useful in the present invention include, without limitation, bicistronic vectors (e.g., pEF-BOS-IRES), plasmid vectors, and adeno-associated virus (AAV) vectors (e.g., pTR-UF5, pTR-UF11, and pTR-UF12).

The nucleic acid construct of the invention is introduced into a eukaryotic cell, in a manner permitting expression of the peptide encoded by the nucleic acid of the invention within the nucleic acid construct of the invention, thereby producing a transformed cell. The eukaryotic cell may be derived from algae, an animal, a plant, a multicellular or other non-yeast fungus, a protozoa, etc. In a particular embodiment, the eukaryotic cell is a plant cell, a mammalian cell, etc.

The nucleic acid construct of the invention may be introduced into the eukaryotic cell by standard methods of transfection or transformation known in the art. Examples of methods by which the construct may be introduced into the cell include, without limitation, electroporation, DEAE Dextran transfection, calcium phosphate transfection, cationic liposome fusion, protoplast fusion, creation of an *in vivo* electrical field, DNA-coated microprojectile bombardment, injection with a recombinant replication-defective virus, homologous recombination, *ex vivo* gene therapy, a viral vector, and naked DNA transfer, or any combination thereof. Recombinant viral vectors suitable for gene therapy include, but are not limited to, vectors derived from the genomes of viruses such as retrovirus, HSV, adenovirus, adeno-associated virus, Semiliki Forest virus, cytomegalovirus, and vaccinia virus.

It is within the confines of the present invention that the nucleic acid construct of the invention may be introduced into the eukaryotic cell *in vitro,* using conventional procedures, to achieve expression in the cells of the fusion protein of the invention. Eukaryotic cells expressing the fusion protein of the invention then may be introduced into a mammal, to provide the mammal with cells such that the functional peptide is expressed within the target organelle in vivo. In such an *ex vivo* gene therapy approach, the eukaryotic cells are preferably removed from the mammal, subjected to DNA techniques to incorporate the nucleic acid construct of the invention, and then reintroduced into the mammal. However, the eukaryotic cells also may be derived from an organism other than the mammal, either of the same, or a different, species.

According to the invention, the nucleic acid construct of the invention is expressed from the nucleus of the eukaryotic cell into which it has been introduced. In fact, the construct is expressed to produce the product of interest in the form of an enzymatically cleavable fusion product in a mitochondria and said fusion protein enters the mitochondria. As used herein, the term "expressed from the nucleus" means that the transcription machinery of the nucleus is used to generate an mRNA transcript of the protein encoded by the nucleic acid of the invention. Thereafter, the mRNA transcript is shuttled to the cytoplasm of the eukaryotic cell, wherein the transcript is translated into a fusion protein. As disclosed herein, the nucleic acid of the invention encoding the MTS is transcribed and translated along with the polynucleotide encoding the product of interest, such that the expressed product bears the MTS. It is this signal that then directs the expressed product of interest in the cytoplasm of the eukaryotic cell to mitochondria. Expression of the peptide may be detected in the eukaryotic cell by detection methods readily determined from the known art, including, without limitation, immunological techniques (e.g., binding studies and Western blotting), hybridization analysis (e.g., using nucleic acid probes), fluorescence imaging techniques, and/or radiation detection. Similarly, the eukaryotic cell may be assayed, using standard protein assays known in the art or disclosed herein, for peptide function.

The present invention may be also particularly useful for treating mitochondrial disorders. Thus, in another aspect, the invention relates to a composition of matter selected from the group consisting of a nucleic acid of the invention, a nucleic acid construct of the invention, a vector of the invention, and a fusion protein of the invention, for use in the treatment of a mitochondrial disorder in an eukaryotic organism, including human beings.

As used herein, a "mitochondrial disorder" is a condition, disease, or disorder characterized by a defect in activity or function of mitochondria, particularly a defect in mitochondrial activity or function that results from, or is associated with, a mutation in mitochondrial DNA (mtDNA). Illustrative, non-limitative, examples of mitochondrial disorders include, without limitation, aging; aminoglycoside-induced deafness; cardiomyopathy; CPEO (chronic progressive external ophthalmoplegia); encephalomyopathy; FBSN (familial bilateral striatal necrosis); KS (Kearns-Sayre) syndrome; LHON (Leber hereditary optic neuropathy); MELAS (mitochondrial myopathy, encephalopathy, lactic acidosis, and stroke-like episodes); MERRF (myoclonic epilepsy with stroke-like episodes); MILS (maternally-inherited Leigh syndrome); mitochondrial myopathy; NARP (neuropathy, ataxia, and retinitis pigmentosa); PEO; SNE (subacute necrotizing encephalopathy). In a particular embodiment of the present invention, the mitochondrial disorder is associated with a mutation (e.g., a point mutation) in mtDNA.

Further, in another aspect, the invention relates to a method for treating a mitochondrial disorder in a subject in need of treatment, comprising introducing to said subject the nucleic acid construct of the invention. The subject may be any eukaryotic organism, including a human being. The mitochondrial disorder may be any of those described above. This method comprises administering to the subject a product of interest for treating a mitochondrial disorder in an amount effective to treat the mitochondrial disorder. The expression "effective to treat the mitochondrial disorder", as used herein, means effective to ameliorate or minimize the clinical impairment or symptoms resulting from the mitochondrial disorder. The amount of peptide effective to treat a mitochondrial disorder in a subject in need of treatment therefore will vary depending on the particular factors of each case, including the type of mitochondrial disorder, the stage of the mitochondrial disorder, the subject's age and weight, the severity of the subject's condition, and the method of administration. These amounts can be readily determined by the skilled artisan, using techniques known in the art and/or disclosed herein. In accordance with the invention, the product of interest may be administered to the subject by introducing into one or more cells of the subject a nucleic acid construct of the invention encoding the product of interest, in a manner permitting expression of the product of interest in the form of a fusion protein. Methods for carrying out this aspect of the present invention are described above. Without limitation, the nucleic acid construct of the invention encoding the product of interest may be introduced into the cells of the subject (either *in situ* in the subject or *ex vivo*) by standard methods of transfection or transformation known in the art, including electroporation, DEAE Dextran transfection, calcium phosphate transfection, cationic liposome fusion, protoplast fusion, creation of an *in vivo* electrical field, DNA-coated microprojectile bombardment, injection with a recombinant replication-defective virus, homologous recombination, *ex vivo* gene therapy, a viral vector, and naked DNA transfer, or any combination thereof. Recombinant viral vectors suitable for gene therapy include, but are not limited to, vectors derived from the genomes of viruses such as retrovirus, HSV, adenovirus, adeno-associated virus, Semiliki Forest virus, cytomegalovirus, and vaccinia virus.

In another aspect, the invention relates to a pharmaceutical composition comprising a nucleic acid of the invention, or a nucleic acid construct of the invention, or a vector of the invention, or a fusion protein of the invention, together with a pharmaceutically acceptable vehicle.

The pharmaceutically acceptable vehicle must be "acceptable" in the sense of being compatible with the other ingredients of the composition, and not deleterious to the recipient thereof. Examples of acceptable pharmaceutical carriers include carboxymethyl cellulose, crystalline cellulose, glycerin, gum arabic, lactose, magnesium stearate, methyl cellulose, powders, saline, sodium alginate, sucrose, starch, talc, and water, among others. Formulations of the pharmaceutical composition may be conveniently presented in unit dosage. The formulations of the present invention may be prepared by methods well-known in the pharmaceutical art. For example, the nucleic acid sequences may be brought into association with a carrier or diluent, as a suspension or solution. Optionally, one or more accessory ingredients (e.g., buffers, flavoring agents, surface active agents, and the like) also may be added. The choice of carrier will depend upon the route of administration. The pharmaceutical composition would be useful for administering the nucleic acid sequences of the present invention to a subject to treat a mitochondrial disorder.

In another aspect, the invention relates to a mitochondria comprising a fusion protein of the invention or a product of interest resulting from the enzymatic cleavage into the mitochondria of said fusion protein. The accumulation of a product of interest within the mitochondria allows for the use of the mitochondria as a vehicle to deliver the product of interest to a cell or host system which has not previously been exposed to the mitochondria containing the product of interest. This delivery can be used to produce an immune reaction in the newly exposed cell or host system, providing a means of effectively and efficiently producing an immune reaction for the generation of antibodies or vaccines. Further, an isolated mitochondria having a product of interest within the mitochondria may be used in assays where the product of interest has an activity which can be measured with or without releasing the product of interest from the mitochondria. Further, a mitochondria having a product of interest within said mitochondria may be purified and transferred into a host cell or host system with the aim of regaining a biological function lost due to a dysfunctional or impaired gene or gene product. Thus, the use of the MTS allows for the production of the MTS alone or fused to a product of interest either in a host cell, host system or in a synthetic manner which does not contain mitochondria. The MTS and/or the fusion product being purified and used as a means to deli

In another aspect, the invention relates to a peptide comprising SEQ ID NO: 1. In a particular embodiment, the MTS is a peptide of formula (I)

$$(X1)_n\text{-RRRA-}(X2)_m \quad (I)$$

wherein

X1 is RL, RAA or RA, wherein A is alanine, L is leucine, and R is arginine;

X2 is ARSLARSAARR, ARR or RR, wherein A is alanine, L is leucine, and R is arginine are those previously defined, and S is serine; and

m and n are, independently, 0 or 1.

In another particular embodiment, the MTS is a peptide whose amino acid sequence is shown in SEQ ID NO: 2, SEQ ID NO: 3 or SEQ ID NO: 4.

Said peptide, which may be used as a MTS, may be produced synthetically or within a living host system and is subsequently purified and fused by methods known to those skilled in the art to a solid support. Said solid support may include but not be limited to capillary columns, protein chips, resins, membranes, etc. The solid support fused MTS having the ability to bind to the mitochondria via interactions with the membrane or membrane proteins and effectively anchoring the mitochondria to the solid support. The MTS fused to the solid support thus providing a means of purifying mitochondria.

The technology provided by the present invention has a lot of advantages. Briefly, the utilisation of the MTS allows for the production of a product of interest, said product of interest being transported via the MTS to the mitochondria of the host cell or host system where it accumulates within the same. Said accumulation allows for the product of interest to be protected from the cellular environment and to protect the host cell or host system from any possible effects the product of interest may have on the host cell or host system. Further, said accumulation also allows for the correct folding of the product of interest since in the case of there being present any unfolded protein within the mitochondria the result is the transcriptional up-regulation of nuclear genes encoding mitochondrial stress proteins such as chaperonin 60 and chaperonin 10. So that even in the case of there being unfolded proteins present, the mitochondrion responds by producing more chaperones to aid in the folding process.

Further, the use of the MTS allows not only for the accumulation of the product of interest within the mitochondria, but the isolation of the mitochondrial fraction from the host cell or host system concomitantly results in the enrichment of the product of interest. The isolation of the mitochondria and enrichment of the product of interest is effectively an initial step in a purification process, one which is common to all products of interest targeted to the mitochondria as the target of this purification step is not the product of interest *per se* but the mitochondria.

In addition, the utilisation of the MTS allows for the accumulation of a product of interest within mitochondria and its accumulation within the mitochondria allows for the use of the mitochondria as a vehicle to deliver the product of interest to a cell or host system which has not previously been exposed to the mitochondria containing the product of interest. This delivery can be used to produce an immune reaction in the newly exposed cell or host system, providing a means of effectively and efficiently producing an immune reaction for the generation of antibodies or vaccines.

Further, the use of the MTS allows for the isolation of mitochondria once said mitochondria have been liberated from the intact cells. The MTS provided by the instant invention may be produced in a host cell, host system or in a synthetic manner and fused to a solid support. The solid support allowing the capture of the mitochondria upon passage and contact with the mitochondria targeting sequence, effectively allowing for the purification of the mitochondria.

The invention is further illustrated but not limited by the following example.

**EXAMPLE 1**

**Production of recombinant red fluorescent protein in a model eukaryotic system (yeast)**

The model eukaryotic system chosen for the successful production of a red fluorescent protein (RFP) was the yeast *Kluyveromyces lactis,* with many features important to higher eukaryotes conserved in yeast [DNA replication, RNA synthesis and processing, protein synthesis, protein targeting to organelles and their translocation across membranes, nuclear pores/ mitochondria/ ER/ peroxisomes etc., secretion, respiration, signal transduction (Ras from humans complements ras mutants in yeast), cytoskeleton and cell cycle regulation].

Various mitochondrial targeting sequences (MTS) were engineered using currently available software to serve as fusion partners; the MTS fraction was then specifically removed by internal mitochondrial proteases at a cleavable protease site at the C-terminus of the MTS. All of this results in the expression, transport and accumulation of the RFP inside the mitochondria of the host system when fused to the synthetically designed and optimised MTS. Following purification/enrichment via differential centrifugation steps the enriched RFP was validated by sequencing the N-terminal of the protein to confirm sequence integrity. Also presented is the purification and characterization of the RFP with special interest in the co-localisation of the fluorescence with fluorescent mitochondrial specific markers.

**Experimental Procedures & Results**

**Production of fusion-gene and associated vectors**

The newly designed and optimised MTS was fused to a synthetic gene for a red fluorescent protein (RFP/ DsRed (Discosoma sp. Red *(red fluorescence marker used in biological microscopy)*). The nucleotide sequence encoding said RFP is shown in SEQ ID NO: 14 and the amino acid sequence of RFP is shown in SEQ ID NO: 15.

The resulting fusion contained the MTS and the RFP in one seamless sequence, the MTS concomitantly containing a cleavage site incorporated which is recognised by the internal processing machinery of the mitochondria, and the fusion gene is incorporated in the yeast transformation vector as described below. The MTS-RFP sequence was generated by PCR from the original vector (pTagRFP-mito) [Evrogen, Cat No: FP147], the PCR product was generated pairing the forward (Fwd) primer for each respective sequence [SEQ ID NO: 9 (PCR forward primer for cloning (MIS-VII)-RFP); SEQ ID NO: 10 (PCR forward primer for cloning (MIS-XIII)-RFP); or SEQ ID NO: 11 (PCR forward primer for cloning (MIS-XIV)-RFP)] with the RFP Reverse Primer [SEQ ID NO: 12 [common PCR reverse primer]. The PCR product was digested by HindIII and NotI and inserted into the expression vector pKLAC2 [New England Biolabs (NEB), Cat No: N3742S)] at the HindIII and NotI sites (removing the factor which is used for secretion). This vector was chosen as it allows the option of cloning to include or exclude the secretion factor which is used for the purpose of secreting proteins via the Golgi apparatus in this model system. In the case of the MTS-RFP fusion sequence the secretion factor is excluded and so any protein expressed should not be secreted via the Golgi apparatus. The vector also contains all the required regulatory elements for expression. Figure 1 shows the vectors so generated which were identified as pMIS-VII-RFP, pMIS-XIII-RFP and pMIS-XIV-RFP, respectively.

Two control plasmids were generated. The first control plasmid, identified as pPOS-CONTROL, as a positive control to send the RFP to the mitochondria, using the primers shown in SEQ ID NO: 13 (PCR forward primer for cloning of the cytochrome-c) and SEQ ID NO: 12 [PCR reverse primer for cloning of RFP], for the cloning of the cytochrome-c-RFP. The MTS-RFP sequence was generated by PCR from the original vector (pTagRFP-mito) [Evrogen, Cat No: FP147]. The PCR product and the vector were digested with HindIII and NotI and the PCR product inserted into the expression vector pKLAC2 [NEB, Cat No: N3742S] at the HindIII and NotI sites to generate the pPOS-CONTROL plasmid. The secretion factor is eliminated and therefore the secretion. The completed vector pPOS-CONTROL is presented in Figure 2.

The second control plasmid, identified as pNEG-CONTROL, is of the RFP protein cloned so as to be secreted, i.e. placed after the secretion factor. The original vector for the RFP sequence (pTagRFP-mito) [Evrogen, Cat No: FP147] was digested with NcoI and NotI enzymes as was the destination (expression) vector (pKLAC2) [NEB], the resulting RFP fragment contained the subunit VIII of human cytochrome C oxidase and the RFP protein sequence. This fragment of DNA was cloned into the NcoI/NotI digested pKLAC2, the resulting RFP containing an additional 7 amino acids at the N-terminal between the secretion signal and the mature product; after processing by the Golgi apparatus for secretion. The resulting vector pNEG-CONTROL is shown in Figure 3.

After cloning the vectors were transformed into the commercially available *K. lactis* [NEB, Cat No: C1001S] cells via methods provided by the manufacturer.

**Stable *K. lactis* transformation and selection**

Linearised vectors were transformed into competent *K.lactis* cells following standard protocols; briefly, the linear DNA was introduced into cells via chemical transformation, the transformants were selected by growing on Yeast Carbon Base media with acetamide as a sole source of nitrogen. The plates also contained 0.6 M KCl and 15 mM CsCl which aids in the selection process by vastly reducing the level of background (non-transformed) growth. Selection was complete after two rounds of selection on this chemically enhanced minimal media.

**Expression of protein and subsequent analysis**

Proteins [cytochrome-c, (MIS-VII)-RFP, (MIS-XIII)-RFP and (MIS-XIV)-RFP] were expressed by growing the transformed and selected cells in an appropriate liquid media containing 4% galactose and 2% glycerol for a period of 48 hours at 30°C with constant shaking at 200 rpm. The RFP shows a red/pink colour after expression in both the mitochondrial targeting and pPOS-CONTROL samples, the RFP secretion control did not display any colour. The cellular and the media fractions were analysed for the presence of the RFP protein in the pellet or the media fraction via Western blot.

The results obtained showed that the proteins [cytochrome-c, (MIS-VII)-RFP, (MIS-XIII)-RFP and (MIS-XIV)-RFP] were accumulated in mitochondrion within the host system (*K. lactis*), the expression of said proteins having little or no effect on the growth of the host system (Table 3).

**Table 3**

| Culture density after protein expression | |
|---|---|
| **Sample** | **O.D. $A_{600}$ (1:100)** |
| CYTOCHROME-C | 0.6 |
| (MIS-VII)-RFP | 0.51 |
| (MIS-XIII)-RFP | 0.62 |
| (MIS-XIV)-RFP | 0.54 |

## Extraction and Western blot analysis of DsRed recombinant protein

100 μl of each of the cultures were centrifuged at 10,000 rpm for 5 minutes to pellet the cellular material, the supernatant was removed to a fresh tube for subsequent analysis. To each of the pellet fractions 50 μl of lysis buffer (0.1 M NaOH pH 10, 2% sodium dodecyl sulfate (SDS), 2 M thiourea, 50 mM EDTA and 5 mM Tris [2-carboxyethyl] phosphine (TCEP)] was added and heated to 95°C for 15 minutes, then 2.5 μl of 4M acetic acid is added and incubated at 95°C for a further 15 minutes. The homogenates were then centrifuged at 13,000 rpm for 10 minutes to pellet any insoluble material. Total soluble protein was quantified by absorbance at 280 nM and 25 μg of total protein was loaded from each sample and separated on a 15% SDS-polyacrylamide gel and transferred to nitrocellulose membranes (0.22 μM) using a semidry apparatus. Membranes were incubated with an anti-RFP antibody from Evrogen [Anti-tRFP Antibody, Cat No: AB234] (dilution 1/10,000) and after washing were then incubated with alkaline phosphatase conjugated secondary antibodies [Promega Anti-Rabbit IgG (Fc) AP Congugate, Cat No: S373B] (dilution 1/10,000). Immunoreactive bands were detected by BCIP/NBT (5-bromo-4-chloro-3-indolyl phosphate/nitro blue tetrazolium) tablets, representing a colorimetric precipitating substrate for the detection of alkaline phosphatase activity.

## Co-Localisation Ability and Analysis of Mitochondria Targeting Sequences (MTS)

Co-localisation analysis is the defining means by which we can positively say that the synthetically designed MTS are performing as they should be. The mitochondria of the yeast will be marked by a specific fluorescent label (MitoTracker Green (MTG) from Invitrogen) and the fluorescent label will be excited to emit the fluorescence which will be detected, the RFP will also be excited and the images overlaid to allow for co-localisation analysis. The co-localisation analysis was performed by the ImageJ software and the Manders and Pearsons coefficients presented.

Cultures containing constructs of the MIS sequences (VII, XIII and XIV) and the control sample [pPOS-CONTROL (mitochondrial accumulation)] were grown in 2 ml of liquid YP (Yeast Peptone) media (composed of 10g of yeast extract and 20g peptone per litre) containing 4% galactose and 1% glycerol at 30°C with 200 rpm constant shaking for 48 hours. After the incubation period the cultures were analysed by taking the optical density (O.D.) at 600 nm to quantify the number of cells per culture. An O.D. value of 1 equates to $3 \times 10^7$ cells per ml of culture. For each culture $5 \times 10^7$ cells were used for the co-localisation analysis. Each sample of cells were first centrifuged at 10,000 rpm for 2 minutes to pellet the cells and the supernatant was removed and the cells re-suspended in 1 ml of 10 mM HEPES buffer pH 7.4, containing 5% glucose. Then Mitotracker Green (Mitotracker Green, MTG) was added to a final concentration of 100 nM (following manufacturer's instructions) and incubated at 30°C for 30 minutes with 100 rpm constant shaking. The cells were centrifuged at 10,000 rpm for 2 minutes and the supernatant removed, the pellet was then washed in 2 ml of 10 mM HEPES buffer pH 7.4 and centrifuged again at 10,000 rpm. The supernatant was removed and the pellet washed for a final time in 2 ml of 10 mM HEPES buffer pH 7.4. The sample was then centrifuged at 10,000 rpm for 2 minutes and the pellet re-suspended in 100 μl of 10 mM HEPES buffer pH 7.4. The samples were then analysed on the LEICA TCS-SP2, the RFP was excited by a laser at 543 nm and the MTG by a laser at 488 nm. The degree of co-localisation is determined after ImageJ analysis with both Manders and Pearsons being shown but the overlap coefficient according to Manders indicates an overlap of the signals and thus represents the true degree of co-localisation. This coefficient is not sensitive to the limitations of typical fluorescence imaging, such as efficiency of hybridization, sample photobleaching, and camera quantum efficiency. The results are shown in Figure 4.

ImageJ software was used to determine the true degree of co-localisation via Manders analysis where the results are from 0 to 1, 0 having no co-localisation and 1 having 100%, and the results are presented in Figure 5.

**Enrichment of Target Protein in the Mitochondrial Fraction by Differential Centrifugation**

Fresh colonies of yeast containing pPOS-CONTROL, MIS-VII-RFP, MIS-XIII-RFP and MIS-XIV-RFP plasmids were grown for a period of 48 hours in 2 ml of liquid YP media in the presence of 4% galactose with 200 rpm constant shaking at a temperature of 30°C. The O.D. A600 of the culture was noted (every 1 O.D. value equates to about 30 $x10^6$ cells). A 200 μl aliquot of the culture was removed and centrifuged at 10,000 rpm for 2 minutes and the supernatant was removed. The pellet was resuspended in 200 μl of PBS containing 1% SDS and 1 mM DTT (dithiothreitol). This was heated at 70°C for 30 minutes and then centrifuged at 13,000 rpm for 10 minutes to pellet insoluble material and the supernatant transferred to a fresh tube for analysis. The remainder of the culture was centrifuged at 10,000 rpm for 5 minutes to collect the cell, the supernatant was removed and the pellet resuspended in 500 μl of 10 mM HEPES pH 7.4 and 5% glucose. 200 Units of lyticase was added and the O.D. A600 was noted at the start of the incubation period. The suspension was incubated at 30°C with 150 rpm constant shaking until the O.D. A600 reduced by 30% of the original. When the 30% decrease was achieved, the suspension was centrifuged at 10,000 rpm for 5 minutes to pellet the cells and the weight of the pellet noted. The cells were then resuspended in 250 μl of 10 mM HEPES pH 7.4 containing 1 mM DTT, 1 M sorbitol and 1 mM EDTA (ethylenediaminetetraacetic acid). To the resuspended cells, 2 g of glass beads (0.5 mm diameter) per 1 g of cells was added. This suspension was vortexed at full speed for 30 seconds and then left to rest on ice for 2 minutes. The process was repeated 4 times in total. The suspension was then centrifuged at 1,500 rpm for 10 minutes to pellet intact cells and heavy organelles. The supernatant was carefully removed and transferred to a fresh tube. The pellet fraction was resuspended in 500 μl of PBS containing 1% SDS and 1 mM DTT which was heated at 70°C for 30 minutes and then centrifuged at 13,000 rpm for 10 minutes to pellet insoluble material and the supernatant transferred to a fresh tube for analysis. The supernatant fraction was centrifuged at 13,000 rpm for 30 minutes to pellet the mitochondrial fraction and the supernatant removed to a fresh tube for subsequent analysis. The pellet was resuspended in 100 μl of PBS containing 1% SDS and 1 mM DTT which was heated at 70°C for 30 minutes and then centrifuged at 13,000 rpm for 10 minutes to pellet insoluble material and the supernatant transferred to a fresh tube for analysis. The protein concentration from each fraction was assayed via the Bradford method, the fractions were analysed on Western blot with 750 ng of protein total loaded for each sample. The primary antibody was the anti-tRFP from Evrogen at 1:15,000 and the secondary antibody was the alkaline phosphatase fused anti-rabbit antibody from Promega at 1:15,000. The results of the images analysed via ImageJ software are presented in Table 4.

**Table 4**

| Enrichment after differential centrifugation | | | | |
|---|---|---|---|---|
| **POS-CONTROL** | **AREA** | **MEAN** | **DENSITY** | **Ratio (With respect to total)** |
| **TOTAL** | 2375 | 48 | 113181 | **1,00** |
| **P1** | 2375 | 8 | 19156 | **0,17** |
| **S1** | 2375 | 33 | 78596 | **0,69** |
| **P2** | 2375 | 58 | 138239 | **1,22** |
| **S2** | 2375 | 16 | 38352 | **0,34** |
| **MIS-VII** | **AREA** | **MEAN** | **DENSITY** | **Ratio (With respect to total)** |
| **TOTAL** | 3328 | 45 | 149797 | **1,00** |
| **P1** | 3328 | 29 | 97782 | **0,65** |
| **S1** | 3328 | 52 | 174271 | **1,16** |
| **P2** | 3328 | 66 | 220617 | **1,47** |
| **S2** | 3328 | 49 | 161706 | **1,08** |
| **MIS-XIII** | **AREA** | **MEAN** | **DENSITY** | **Ratio (With respect to total)** |
| **TOTAL** | 3456 | 34 | 116430 | **1,00** |
| **P1** | 3456 | 26 | 89542 | **0,77** |
| **S1** | 3456 | 40 | 138729 | **1,19** |

(continued)

| MIS-XIII | AREA | MEAN | DENSITY | Ratio (With respect to total) |
|---|---|---|---|---|
| **P2** | 3456 | 64 | 220708 | **1,90** |
| **S2** | 3456 | 36 | 125069 | **1,07** |
| **MIS-XIV** | **AREA** | **MEAN** | **DENSITY** | **Ratio (With respect to total)** |
| **TOTAL** | 3090 | 62 | 190768 | **1,00** |
| **P1** | 3090 | 44 | 137280 | **0,72** |
| **S1** | 3090 | 62 | 190117 | **1,00** |
| **P2** | 3090 | 89 | 274554 | **1,44** |
| **S2** | 3090 | 62 | 193008 | **1,01** |

where:
T - Total
S1 - Supernatant from the 1500rpm centrifugation
P1 - Pellet from the 1500rpm centrifugation
P2 - Pellet from the 13,000rpm centrifugation
S2 - Supernatant from the 13,000rpm centrifugation

The enriched sample was analysed on a 15% SDS-PAGE gel and stained with Coomassie blue. After destaining the gel a piece of the SDS-PAGE gel was cut at the position where the RFP was expected. This was washed extensively in water and analysed by MALDI-TOF-TOF analysis after trypsin digestion. The analysis revealed that for the MIS-VII, MIS-XIII and MIS-XIV constructions the cleavage was correct and that the RFP was present without the MTS, the removal of the MTS was performed by the internal machinery of the mitochondria and confirmed that cleavage was correct leaving the RFP in the mature form (removing the MTS) and is presented in Table 5 MALDI-TOF-TOF sequence confirmation.

**Table 5**

| MALDI-TOF-TOF sequence confirmation | | | |
|---|---|---|---|
| **Mitochondrial Targeting Sequence (MTS)** | **RFP sequence (1-9)** | **Peptide detected** | **Position** |
| MIS-VII | VSKGEELIK | VSKGEELIK | 1-9 |
| MIS-XIII | VSKGEELIK | VSKGEELIK | 1-9 |
| MIS-XIV | VSKGEELIK | VSKGEELIK | 1-9 |

**Structure analysis of the central CDE**

The central Core Domain Element (CDE) [SEQ ID NO: 1 (RRRA)], which is present in all the MTS previously designed, has been demonstrated capable of forming a helical structure, the helical structure which aids in the process of transport to the mitochondria. Analysis of this sequence by NetSurfP (http://www.cbs.dtu.dk/services/NetSurfP/) demonstrated that helical nature of the sequence, the results of which are shown in Table 6.

**Table 6**

| Core Domain Element helical confirmation | | |
|---|---|---|
| **Sequence name and amino acid** | **Amino Acid Position** | **Helix Probability score (0-1)** |
| **MIS-VII (SEQ ID NO: 2)** | | |
| R | 3 | 0,717 |
| R | 4 | 0,858 |
| R | 5 | 0,938 |
| A | 6 | 0,923 |

(continued)

| MIS-XIII (SEQ ID NO: 3) | | |
|---|---|---|
| R | 4 | 0,782 |
| R | 5 | 0,879 |
| R | 6 | 0,879 |
| A | 7 | 0,879 |
| **MIS-XIV (SEQ ID NO: 4)** | | |
| R | 3 | 0,622 |
| R | 4 | 0,714 |
| R | 5 | 0,717 |
| A | 6 | 0,751 |

**References:**

[1] Genetic Engineering and Biotechnology News, Jan 1 2006 (Vol. 26, No. 1)

[2] BioProcess International 5(6):22-28 (June 2007)

[3] Targeting and retention of HPV16 E7 to the endoplasmic reticulum enhances immune tumor protection. Loera-Arias MJ, Martínez-Pérez AG, Barrera-Hernández A, Ibarra-Obregón ER, González-Saldívar G, Martínez-Ortega JI, Rosas-Taraco A, Villanueva-Olivo A, Esparza-González SC, Villatoro-Hernandez J, Saucedo-Cárdenas O, Montes-de-Oca-Luna R. J Cell Mol Med. 2009 Oct 10. [Epub ahead of print]

[4] Expression and purification of moricin CM4 and human beta-defensins 4 in Escherichia coli using a new technology. Shen Y, Ai HX, Song R, Liang ZN, Li JF, Zhang SQ, Microbiol Res. 2010 Jan 18. [Epub ahead of print]

[5] Markley N. Nykiforuk C, Boothe J, Moloney M, Producing Proteins Using Transgenic Oilbody-OleosinTechnology, BIOPHARM International, June 2006.

[6] Arnau J, Lauritzen C, Petersen GE, Pedersen J (2006) Current strategies for the use of affinity tags and tag removal for the purification of recombinant proteins. Protein Expr Purif 48: 1-13

[7] Shen A, Lupardus PJ, Morell M, Ponder EL, Sadaghiani AM, et al. (2009) Simplified, Enhanced Protein Purification Using an Inducible, Autoprocessing Enzyme Tag. PLoS ONE 4(12): e8119. doi:10.1371/journal.pone.0008119

[8] Genetic Engineering News (Volume 27, Number 11, June 2007)

[9] Ikonomou L, Schneider Y.-J., Agathos S. N., Insect cell culture for industrial production of recombinant proteins, Applied Microbiology and Biotechnology, Volume 62, Issue 1, p1-20, 2003

[10] Klein M, Binder S and Brennicke A, Purification of Mitochondria from Arabidopsis, Methods in Molecular Biology, Volume 82, p49-53

[11] Bright monomeric red fluorescent protein with an extended fluorescence lifetime. Merzlyak EM, Goedhart J, Shcherbo D, Bulina ME, Shcheglov AS, Fradkov AF, Gaintzeva A, Lukyanov KA, Lukyanov S, Gadella TW, Chudakov DM, Nat Methods (2007) 4:555-557

[12] Claros, M. G. and Vincens, P. (1996). Computational method to predict mitochondrially imported proteins and their targeting sequences. Eur. J. Biochem. 241, 779-786

[13] Rizzuto R, Brini M, Pizzo P, Murgia M, Pozzan T. Chimeric green fluorescent protein as a tool for visualizing subcellular organelles in living cells. Curr Biol. 1995; 5 (6):635-42

[14] Hammen, P. K., Gorenstein, D. G., and Weiner, H. (1994) Biochemistry 33, 8610-8617

[15] A mitochondrial specific stress response in mammalian cells, The EMBO Journal (2002) 21, 4411- 4419

[16] Klaus, C., Guiard, B., Neupert, W. and Brunner, M. (1996) Determinants in the presequence of cytochrome b2 for import into mitochondria and for proteolytic processing. Eur. J. Biochem. 236, 856-861

[17] Ogishima, T., Niidome, T., Shimokata, K., Kitada, S. and Ito, A. (1995) Analysis of elements in the substrate required for processing by mitochondrial processing peptidase. J. Biol. Chem. 270, 30322-30326

[18] Niidome, T., Kitada, S., Shimokata, K., Ogishima, T. and Ito, A. (1994) Arginine residues in the extension peptide are required for cleavage of a precursor by mitochondrial processing peptidase. Demonstration using synthetic peptide as a substrate. J. Biol. Chem. 269, 24719-24722

[19] Schneider, G., SjoÈ ling, S., Wallin, E., Wrede, P., Glaser, E. and von Heijne, G. (1998) Feature-extraction from endopeptidase cleavage sites in mitochondrial targeting peptides. Proteins, 30, 49-60
[20] von Heijne, G., Steppuhn, J. and Herrmann, R.G. (1989) Domain structure of mitochondrial and chloroplast targeting peptides. Eur. J. Biochem. 180, 535-545
[21] Arretz, M., Schneider, H., Guiard, B., Brunner, M. and Neupert, W. (1994) Characterization of the mitochondrial processing peptidase of Neurospora crassa. J. Biol. Chem. 269, 4959-4967
[22] M Cregg, K J Barringer, A Y Hessler, and K R Madden. Pichia pastoris as a host system for transformations. Mol Cell Biol. 1985 December; 5(12): 3376-3385.

## Claims

**1.** A nucleic acid sequence comprising:

a first polynucleotide containing a nucleotide sequence that encodes a mitochondria-targeting sequence (MTS), wherein said MTS comprises the amino acid sequence shown in SEQ ID NO: 1, and is capable of directing the protein towards a mitochondria;
a second polynucleotide containing a nucleotide sequence that encodes an amino acid sequence that is specifically cleavable by a mitochondrial protease enzyme; and
a third polynucleotide containing the nucleotide sequence that encodes a product of interest;

wherein the 3' end of said first polynucleotide is linked to the 5' end of said second polynucleotide and the 3' end of said second polynucleotide is linked to the 5' end of said third polynucleotide.

**2.** Nucleic acid sequence according to claim 1, wherein the MTS is a peptide of formula (I)

$$(X1)_n\text{-RRRA-}(X2)_m$$

(I)

wherein
X1 is RL, RAA or RA, wherein A is alanine, L is leucine, and R is arginine;
X2 is ARSLARSAARR, ARR or RR, wherein A is alanine, L is leucine, and R is arginine are those previously defined, and S is serine; and
m and n are, independently, 0 or 1.

**3.** Nucleic acid according to claim 2, wherein the MTS is a peptide whose amino acid sequence is shown in SEQ ID NO: 2, SEQ ID NO: 3 or SEQ ID NO: 4.

**4.** A nucleic acid construct comprising (i) a nucleic acid according to any of claims 1 to 3, and (ii) a transcriptional regulatory element that regulates the transcription of said nucleic acid (i).

**5.** A vector comprising a nucleic acid according to any of claims 1 to 3, or a nucleic acid construct according to claim 4.

**6.** A fusion protein comprising:

an amino acid sequence comprising a mitochondria-targeting sequence (MTS), wherein said MTS comprises the amino acid sequence shown in SEQ ID NO: 1, and is capable of directing a peptide or protein to which it is bound towards a mitochondria;
an amino acid sequence that is specifically cleavable by a mitochondrial protease enzyme; and
a product of interest;
said fusion protein being the expression product of the nucleic acid of any of claims 1-3.

**7.** A eukaryotic host cell comprising a nucleic acid according to any of claims 1 to 3, or a nucleic acid construct according to claim 4, or a vector according to claim 5, or a fusion protein according to claim 6.

**8.** A transgenic host system, said host system comprising, integrated in its genome a nucleic acid according to any of claims 1 to 3.

**9.** A method for producing a product of interest, which comprises:

a) growing a eukaryotic host cell according to claim 7, under conditions that allow the production and expression of said product of interest in the form of an enzymatically cleavable fusion protein in a mitochondria, and, if desired,
b) purifying the product of interest from the mitochondria;

or alternatively

a) transforming a host system with a nucleic acid construct according to claim 4 to be integrated in the genome of a host system;
b) generating a complete transgenic host system;
c) growing said complete transgenic host system under conditions that allow for the production and expression of the product of interest in the form of an enzymatically cleavable fusion product in a mitochondria; and, if desired
d) purifying said product of interest from the mitochondria.

**10.** A method for delivering a product of interest to a mitochondria, comprising the steps of:

a) introducing a nucleic acid construct according to claim 4 into an eukaryotic cell to produce a transformed cell; and
b) expressing said nucleic acid construct from the nucleus of the transformed cell.

**11.** A pharmaceutical composition comprising a nucleic acid according to any of claims 1 to 3, or a nucleic acid construct according to claim 4, or a vector according to claim 5, or a fusion protein according to claim 6, together with a pharmaceutically acceptable vehicle.

**12.** A composition of matter selected from the group consisting of a nucleic acid according to any of claims 1 to 3, a nucleic acid construct according to claim 4, a vector according to claim 5, and a fusion protein according to claim 6, for use in the treatment of a mitochondrial disorder in an eukaryotic organism.

**13.** A mitochondria comprising a fusion protein according to claim 6 or a product of interest resulting from the enzymatic cleavage into the mitochondria of said fusion protein.

**14.** A peptide of formula (I)

$$(X1)_n\text{-RRRA-}(X2)_m$$

(I)

wherein
X1 is RL, RAA or RA, wherein A is alanine, L is leucine, and R is arginine;
X2 is ARSLARSAARR, ARR or RR, wherein A is alanine, L is leucine, and R is arginine are those previously defined, and S is serine; and
m and n are, independently, 0 or 1.

**15.** Peptide according to claim 14, selected from the group consisting of the peptides whose amino acid sequence is shown in SEQ ID NO: 2, SEQ ID NO: 3 or SEQ ID NO: 4.

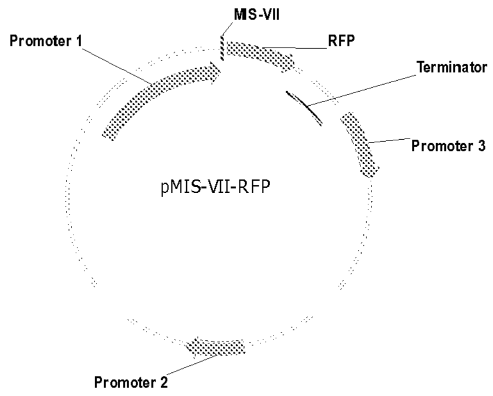
MS-VII
Promoter 1
RFP
Terminator
Promoter 3
pMIS-VII-RFP
Promoter 2

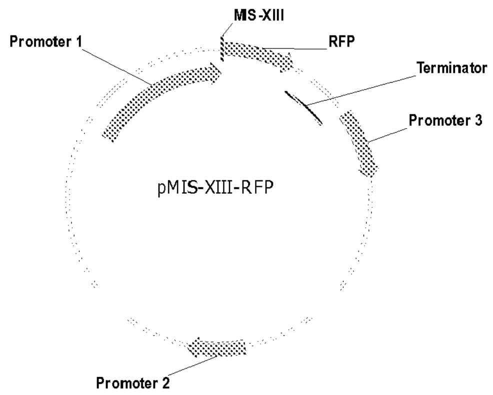
MS-XIII
Promoter 1
RFP
Terminator
Promoter 3
pMIS-XIII-RFP
Promoter 2

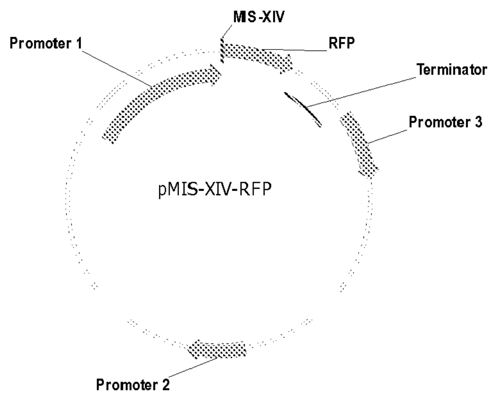
MS-XIV
Promoter 1
RFP
Terminator
Promoter 3
pMIS-XIV-RFP
Promoter 2

**Figure 1**

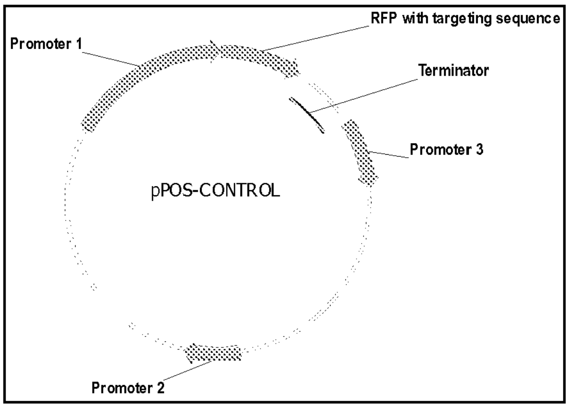


**Figure 2**

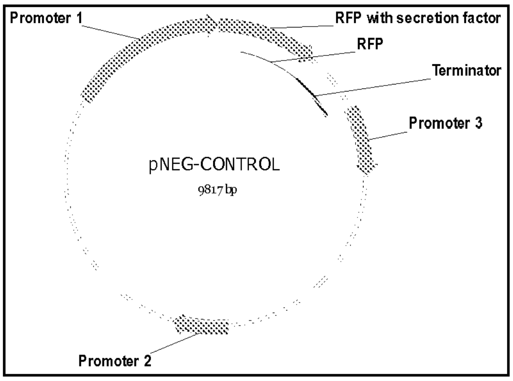


**Figure 3**

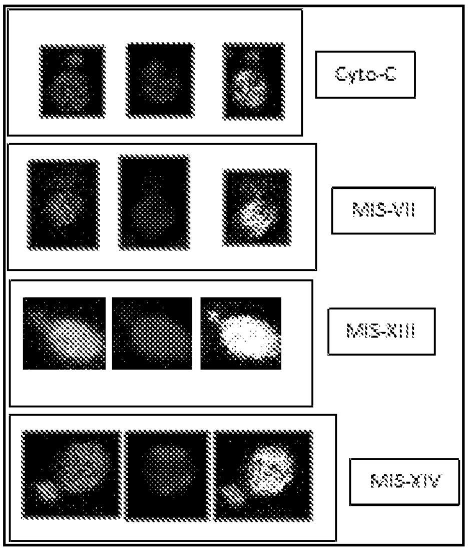


Figure 4

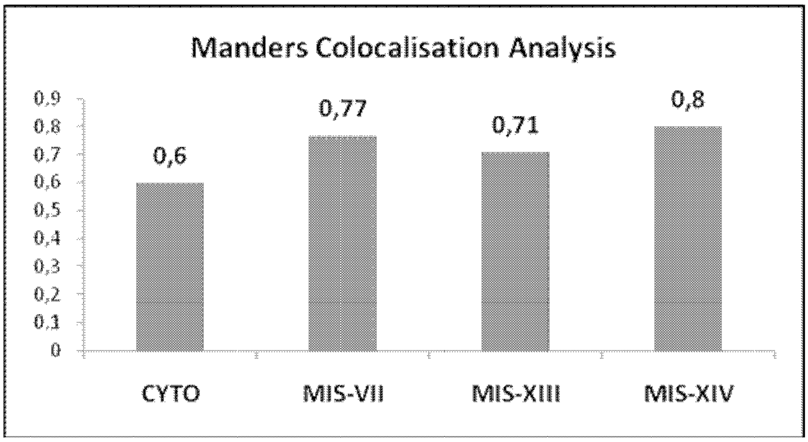


Figure 5

Europäisches Patentamt
European Patent Office
Office européen des brevets

# EUROPEAN SEARCH REPORT

Application Number
EP 10 38 2058

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | US 5 686 264 A (GAYNOR RICHARD B [US] ET AL) 11 November 1997 (1997-11-11)<br>* abstract; figure 1 *<br>* SEQ ID NO:12 is identical to SEQ ID NO:1 *<br>----- | 14 | INV.<br>C12N15/62<br>C12N15/79 |
| Y | KADOMATSU ET AL: "Mitochondrial import of Omi: The definitive role of the putative transmembrane region and multiple processing sites in the amino-terminal segment"<br>BIOCHEMICAL AND BIOPHYSICAL RESEARCH COMMUNICATIONS, ACADEMIC PRESS INC. ORLANDO, FL, US LNKD- DOI:10.1016/J.BBRC.2007.07.053,<br>vol. 361, no. 2,<br>6 August 2007 (2007-08-06), pages 516-521, XP022184886<br>ISSN: 0006-291X<br>* abstract *<br>* page 519, column 2, paragraph 2 - page 520; figure 4 *<br>----- | 1-13,15 | |
| Y | SHOKOLENKO I N ET AL: "TAT-mediated protein transduction and targeted delivery of fusion proteins into mitochondria of breast cancer cells"<br>DNA REPAIR, ELSEVIER, AMSTERDAM, NL LNKD- DOI:10.1016/J.DNAREP.2004.11.009,<br>vol. 4, no. 4, 4 April 2005 (2005-04-04), pages 511-518, XP004752785<br>ISSN: 1568-7864<br>* abstract *<br>* page 516, column 2 - page 517; figures 1-4 *<br>-----<br>-/-- | 1-13,15 | TECHNICAL FIELDS SEARCHED (IPC)<br>C12N |

The present search report has been drawn up for all claims

3

| Place of search | Date of completion of the search | Examiner |
|---|---|---|
| Munich | 8 July 2010 | Mossier, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 38 2058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANG Y ET AL: "The presequence of rat liver aldehyde dehydrogenase requires the presence of an alpha-helix at its N-terminal region which is stabilized by the helix at its C termini" JOURNAL OF BIOLOGICAL CHEMISTRY, AMERICAN SOCIETY FOR BIOCHEMISTRY AND MOLECULAR BIOLOGY, INC, US, vol. 268, no. 7, 1 January 1993 (1993-01-01), pages 4759-4765, XP002972386 ISSN: 0021-9258 * abstract; figure 1 * ----- | 1-13,15 | |
| Y | KNECHT W ET AL: "Rat Dihydroorotate Dehydrogenase: Isolation of the Recombinant Enzyme from Mitochondria of Insect Cells" PROTEIN EXPRESSION AND PURIFICATION, ACADEMIC PRESS, SAN DIEGO, CA LNKD- DOI:10.1006/PREP.1996.0714, vol. 10, no. 1, 1 June 1997 (1997-06-01), pages 89-99, XP004451804 ISSN: 1046-5928 * abstract * * page 93, column 1 * * page 94, column 2, paragraph 3 - page 95, column 2, paragraph 2 * ----- | 1-13,15 | TECHNICAL FIELDS SEARCHED (IPC) |
| Y | WO 00/28041 A1 (MICROBIOLOGICAL RES AUTHORITY [GB]; SHONE CLIFFORD CHARLES [GB]; SUTTO) 18 May 2000 (2000-05-18) * abstract * * page 15, line 12 - line 25; figure 1 * ----- -/-- | 1-13,15 | |

The present search report has been drawn up for all claims

| Place of search | Date of completion of the search | Examiner |
|---|---|---|
| Munich | 8 July 2010 | Mossier, Birgit |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 38 2058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2009/098682 A2 (YISSUM RES DEV CO [IL]; HADASIT MED RES SERVICE [IL]; GALSKI-LORBERBOU) 13 August 2009 (2009-08-13) * abstract * * page 4, line 30 - page 6, line 8 * * page 9, line 5 - line 29 * ----- | 1-13,15 | |
| Y | US 2004/072774 A1 (MANFREDI GIOVANNI [US] ET AL) 15 April 2004 (2004-04-15) * abstract * * paragraph [0014] - paragraph [0024] * * paragraph [0039] * * figure 1; examples 1-6 * ----- | 1-13,15 | |
| A | WO 2007/149946 A2 (INVITROGEN CORP [US]; HANSON GEORGE [US]; THOMPSON DAVID [US]) 27 December 2007 (2007-12-27) * abstract * * paragraph [0071]; example 1 * ----- | 1-15 | |
| A | PFANNER NIKOLAUS ET AL: "Mitochondrial protein import: Two membranes, three translocases" CURRENT OPINION IN CELL BIOLOGY, vol. 14, no. 4, August 2002 (2002-08), pages 400-411, XP002589861 ISSN: 0955-0674 * the whole document * ----- -/-- | 1-15 | TECHNICAL FIELDS SEARCHED (IPC) |

3

The present search report has been drawn up for all claims

| Place of search | Date of completion of the search | Examiner |
|---|---|---|
| Munich | 8 July 2010 | Mossier, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 38 2058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YAMADA YUMA ET AL: "Mitochondrial drug delivery systems for macromolecule and their therapeutic application to mitochondrial diseases." ADVANCED DRUG DELIVERY REVIEWS 2008 OCT-NOV LNKD- PUBMED:18655816, vol. 60, no. 13-14, October 2008 (2008-10), pages 1439-1462, XP25406649 ISSN: 1872-8294 * abstract * ----- | 1-15 | |
| A | OBITA ET AL: "Peptide Library Approach with a Disulfide Tether to Refine the Tom20 Recognition Motif in Mitochondrial Presequences" JOURNAL OF MOLECULAR BIOLOGY, LONDON, GB LNKD- DOI:10.1016/S0022-2836(03)00288-2, vol. 328, no. 2, 25 April 2003 (2003-04-25), pages 495-504, XP005472714 ISSN: 0022-2836 * abstract * ----- | 1-15 | TECHNICAL FIELDS SEARCHED (IPC) |
| A | MUTO T ET AL: "NMR identification of the Tom20 binding segment in mitochondrial presequences" JOURNAL OF MOLECULAR BIOLOGY, LONDON, GB LNKD- DOI:10.1006/JMBI.2000.4397, vol. 306, no. 2, 16 February 2001 (2001-02-16), pages 137-143, XP004466014 ISSN: 0022-2836 * abstract; figure 3 * ----- | 1-15 | |

The present search report has been drawn up for all claims

3

| Place of search | Date of completion of the search | Examiner |
|---|---|---|
| Munich | 8 July 2010 | Mossier, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 38 2058

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2010

| Patent document cited in search report | | Publication date | Patent family member(s) | | | Publication date |
|---|---|---|---|---|---|---|
| US 5686264 | A | 11-11-1997 | US | 5994108 | A | 30-11-1999 |
| WO 0028041 | A1 | 18-05-2000 | AU | 754883 | B2 | 28-11-2002 |
| | | | AU | 1059400 | A | 29-05-2000 |
| | | | CA | 2349496 | A1 | 18-05-2000 |
| | | | EP | 1127136 | A1 | 29-08-2001 |
| | | | JP | 2002529093 | T | 10-09-2002 |
| | | | US | 2005255093 | A1 | 17-11-2005 |
| WO 2009098682 | A2 | 13-08-2009 | NONE | | | |
| US 2004072774 | A1 | 15-04-2004 | NONE | | | |
| WO 2007149946 | A2 | 27-12-2007 | US | 2007292917 | A1 | 20-12-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5874265 A


### Non-patent literature cited in the description


- **SAMBROOK et al.** Molecular cloning, a Laboratory Manual. Cold Spring Harbor Laboratory Press, 1989
- **SAMBROOK et al.** The application of techniques found in Molecular cloning, a Laboratory Manual. Cold Spring Harbor Laboratory Press, 1989
- **TURPEN et al.** *J. Virol. Methods,* 1993, vol. 42, 227-239
- *Genetic Engineering and Biotechnology News,* 01 January 2006, vol. 26 (1
- *BioProcess International,* June 2007, vol. 5 (6), 22-28
- **LOERA-ARIAS MJ ; MARTÍNEZ-PÉREZ AG ; BARRERA-HERNÁNDEZ A ; IBARRA-OBREGÓN ER ; GONZÁLEZ-SALDÍVAR G ; MARTÍNEZ-ORTEGA JI ; ROSAS-TARACO A ; VILLANUEVA-OLIVO A ; ESPARZA-GONZÁLEZ SC ; VILLATORO-HERNANDEZ J.** *J Cell Mol Med.,* 10 October 2009
- **SHEN Y ; AI HX ; SONG R ; LIANG ZN ; LI JF ; ZHANG SQ.** *Microbiol Res.,* 18 January 2010
- **MARKLEY N ; NYKIFORUK C ; BOOTHE J ; MOLONEY M.** Producing Proteins Using Transgenic Oilbody-OleosinTechnology. *BIOPHARM International,* June 2006
- **ARNAU J ; LAURITZEN C ; PETERSEN GE ; PEDERSEN J.** Current strategies for the use of affinity tags and tag removal for the purification of recombinant proteins. *Protein Expr Purif,* vol. 48, 1-13
- **SHEN A ; LUPARDUS PJ ; MORELL M ; PONDER EL ; SADAGHIANI AM et al.** Simplified, Enhanced Protein Purification Using an Inducible. *Autoprocessing Enzyme Tag. PLoS ONE,* 2009, vol. 4 (12), e8119
- *Genetic Engineering News,* June 2007, vol. 27 (11
- **IKONOMOU L ; SCHNEIDER Y.-J. ; AGATHOS S. N.** Insect cell culture for industrial production of recombinant proteins. *Applied Microbiology and Biotechnology,* 2003, vol. 62 (1), 1-20
- **KLEIN M ; BINDER S ; BRENNICKE A.** Purification of Mitochondria from Arabidopsis. *Methods in Molecular Biology,* vol. 82, 49-53
- **MERZLYAK EM ; GOEDHART J ; SHCHERBO D ; BULINA ME ; SHCHEGLOV AS ; FRADKOV AF ; GAINTZEVA A ; LUKYANOV KA ; LUKYANOV S ; GADELLA TW.** *Nat Methods,* 2007, vol. 4, 555-557
- **CLAROS, M. G. ; VINCENS, P.** Computational method to predict mitochondrially imported proteins and their targeting sequences. *Eur. J. Biochem.,* 1996, vol. 241, 779-786
- **RIZZUTO R ; BRINI M ; PIZZO P ; MURGIA M ; POZZAN T.** Chimeric green fluorescent protein as a tool for visualizing subcellular organelles in living cells. *Curr Biol.,* 1995, vol. 5 (6), 635-42
- **HAMMEN, P. K. ; GORENSTEIN, D. G. ; WEINER, H.** *Biochemistry,* 1994, vol. 33, 8610-8617
- A mitochondrial specific stress response in mammalian cells. *EMBO Journal,* 2002, vol. 21, 4411-4419
- **KLAUS, C. ; GUIARD, B. ; NEUPERT, W. ; BRUNNER, M.** Determinants in the presequence of cytochrome b2 for import into mitochondria and for proteolytic processing. *Eur. J. Biochem.,* 1996, vol. 236, 856-861
- **OGISHIMA, T. ; NIIDOME, T. ; SHIMOKATA, K. ; KITADA, S. ; ITO, A.** Analysis of elements in the substrate required for processing by mitochondrial processing peptidase. *J. Biol. Chem.,* 1995, vol. 270, 30322-30326
- **NIIDOME, T. ; KITADA, S. ; SHIMOKATA, K. ; OGISHIMA, T. ; ITO, A.** Arginine residues in the extension peptide are required for cleavage of a precursor by mitochondrial processing peptidase. Demonstration using synthetic peptide as a substrate. *J. Biol. Chem.,* 1994, vol. 269, 24719-24722
- **SCHNEIDER, G. ; SJOÈ LING, S. ; WALLIN, E. ; WREDE, P. ; GLASER, E. ; VON HEIJNE, G.** Feature-extraction from endopeptidase cleavage sites in mitochondrial targeting peptides. *Proteins,* 1998, vol. 30, 49-60
- **VON HEIJNE, G. ; STEPPUHN, J. ; HERRMANN, R.G.** Domain structure of mitochondrial and chloroplast targeting peptides. *Eur. J. Biochem.,* 1989, vol. 180, 535-545

- **ARRETZ, M. ; SCHNEIDER, H. ; GUIARD, B. ; BRUNNER, M. ; NEUPERT, W.** Characterization of the mitochondrial processing peptidase of Neurospora crassa. *J. Biol. Chem.,* 1994, vol. 269, 4959-4967
- **M CREGG ; K J BARRINGER ; A Y HESSLER ; K R MADDEN.** Pichia pastoris as a host system for transformations. *Mol Cell Biol.,* December 1985, vol. 5 (12), 3376-3385

## SEQUENCE LISTING

<110> 3T-Science, S.L.

<120> PRODUCTION OF PEPTIDES AND PROTEINS BY ACCUMULATION IN MITOCHONDRIA

<130> P5625EP00

<160> 16

<170> PatentIn version 3.5

<210> 1
<211> 4
<212> PRT
<213> Artificial Sequence

<220>
<223> Core Domain Element (CDE) of the mitochondrial targeting sequence (MTS)

<400> 1

```
Arg Arg Arg Ala
1
```

<210> 2
<211> 17
<212> PRT
<213> Artificial Sequence

<220>
<223> MTS domain identified as MIS-VII

<400> 2

```
Arg Leu Arg Arg Arg Ala Ala Arg Ser Leu Ala Arg Ser Ala Ala Arg
1               5                   10                  15

Arg
```

<210> 3
<211> 10
<212> PRT
<213> Artificial Sequence

<220>
<223> MTS domain identified as MIS-XIII

<400> 3

```
Arg Ala Ala Arg Arg Arg Ala Ala Arg Arg
1               5                   10
```

<210> 4
<211> 8
<212> PRT
<213> Artificial Sequence

<220>
<223> MTS domain identified as MTS-XIV

<400> 4

Arg Ala Arg Arg Arg Ala Arg Arg
1               5

<210> 5
<211> 12
<212> DNA
<213> Artificial Sequence

<220>
<223> sequence encoding the CDE (SEQ ID NO: 1)

<400> 5
agaagaagag ct 12

<210> 6
<211> 54
<212> DNA
<213> Artificial Sequence

<220>
<223> sequence encoding MIS-VII (SEQ ID NO: 2)

<400> 6
atgagattga gaagaagagc tgctagatct ttcgctagat ctgctgctag aaga 54

<210> 7
<211> 33
<212> DNA
<213> Artificial Sequence

<220>
<223> sequence encoding MIS-XIII (SEQ ID NO: 3)

<400> 7
atgagagctg ctagaagaag agctgctaga aga 33

<210> 8
<211> 27
<212> DNA
<213> Artificial Sequence

<220>

<223> sequence encoding MIS-XIV (SEQ ID NO: 4)

<400> 8
atgagagcta gaagaagagc tagaaga 27

<210> 9
<211> 82
<212> DNA
<213> Artificial Sequence

<220>
<223> PCR forward primer for cloning (MIS-VII)-RFP

<400> 9
gcaagcttat gagattgaga agaagagctg ctagatcttt ggctagatct gctgctagaa 60

gagtgtctaa gggcgaagag ct 82

<210> 10
<211> 61
<212> DNA
<213> Artificial Sequence

<220>
<223> PCR forward primer for cloning (MIS-XIII)-RFP

<400> 10
gcaagcttat gagagctgct agaagaagag ctgctagaag agtgtctaag ggcgaagagc 60

t 61

<210> 11
<211> 55
<212> DNA
<213> Artificial Sequence

<220>
<223> PCR forward primer for cloning (MIS-XV)-RFP

<400> 11
gcaagcttat gagagctaga agaagagcta gaagagtgtc taagggcgaa gagct 55

<210> 12
<211> 30
<212> DNA
<213> Artificial Sequence

<220>
<223> RFP reverse primer

<400> 12
gtcgcggccg ctcaattaag tttgtgcccc 30

<210> 13
<211> 31
<212> DNA
<213> Artificial Sequence

<220>
<223> PCR forward primer for cloning of the cytochrome-c oxidase

<400> 13
gcgcaagctt atgtccgtcc tgacgccgct g 31

<210> 14
<211> 708
<212> DNA
<213> Artificial Sequence

<220>
<223> red fluorescent protein (RFP)

<400> 14
gtgtctaagg gcgaagagct gattaaggag aacatgcaca tgaagctgta catggagggc 60

accgtgaaca accaccactt caagtgcaca tccgagggcg aaggcaagcc ctacgagggc 120

acccagacca tgagaatcaa ggtggtcgag ggcggccctc tcccctcgc cttcgacatc 180

ctggctacca gcttcatgta cggcagcaga accttcatca accacaccca gggcatcccc 240

gacttcttta agcagtcctt ccctgagggc ttcacatggg agagagtcac cacatacgaa 300

gacgggggcg tgctgaccgc tacccaggac accagcctcc aggacggctg cctcatctac 360

aacgtcaaga tcagaggggt gaacttccca tccaacggcc ctgtgatgca gaagaaaaca 420

ctcggctggg aggccaacac cgagatgctg taccccgctg acggcggcct ggaaggcaga 480

agcgacatgg ccctgaagct cgtgggcggg ggccacctga tctgcaactt caagaccaca 540

tacagatcca agaaacccgc taagaacctc aagatgcccg gcgtctacta tgtggaccac 600

agactggaaa gaatcaagga ggccgacaaa gagacctacg tcgagcagca cgaggtggct 660

gtggccagat actgcgacct ccctagcaaa ctggggcaca aacttaat 708

<210> 15
<211> 236
<212> PRT
<213> Artificial Sequence

<220>
<223> red fluorescent protein (RFP)

<400> 15

Val Ser Lys Gly Glu Glu Leu Ile Lys Glu Asn Met His Met Lys Leu
1 5 10 15

```
Tyr Met Glu Gly Thr Val Asn Asn His His Phe Lys Cys Thr Ser Glu
            20                  25                  30

Gly Glu Gly Lys Pro Tyr Glu Gly Thr Gln Thr Met Arg Ile Lys Val
        35                  40                  45

Val Glu Gly Gly Pro Leu Pro Phe Ala Phe Asp Ile Leu Ala Thr Ser
    50                  55                  60

Phe Met Tyr Gly Ser Arg Thr Phe Ile Asn His Thr Gln Gly Ile Pro
65                  70                  75                  80

Asp Phe Phe Lys Gln Ser Phe Pro Glu Gly Phe Thr Trp Glu Arg Val
                85                  90                  95

Thr Thr Tyr Glu Asp Gly Gly Val Leu Thr Ala Thr Gln Asp Thr Ser
            100                 105                 110

Leu Gln Asp Gly Cys Leu Ile Tyr Asn Val Lys Ile Arg Gly Val Asn
        115                 120                 125

Phe Pro Ser Asn Gly Pro Val Met Gln Lys Lys Thr Leu Gly Trp Glu
    130                 135                 140

Ala Asn Thr Glu Met Leu Tyr Pro Ala Asp Gly Gly Leu Glu Gly Arg
145                 150                 155                 160

Ser Asp Met Ala Leu Lys Leu Val Gly Gly Gly His Leu Ile Cys Asn
                165                 170                 175

Phe Lys Thr Thr Tyr Arg Ser Lys Lys Pro Ala Lys Asn Leu Lys Met
            180                 185                 190

Pro Gly Val Tyr Tyr Val Asp His Arg Leu Glu Arg Ile Lys Glu Ala
        195                 200                 205

Asp Lys Glu Thr Tyr Val Glu Gln His Glu Val Ala Val Ala Arg Tyr
    210                 215                 220

Cys Asp Leu Pro Ser Lys Leu Gly His Lys Leu Asn
225                 230                 235
```

```
<210>  16
<211>  29
<212>  PRT
<213>  Artificial Sequence

<220>
<223>  Cleavage site of E1alpha pyruvate dehydrogenase

<400>  16

Met Arg Lys Met Leu Ala Ala Val Ser Arg Val Leu Ser Gly Ala Ser
1               5                   10                  15

Gln Lys Pro Ala Ser Arg Val Leu Val Ala Ser Arg Asn
            20                  25
```